# EUROPEAN PATENT APPLICATION

(11) **EP 2 135 871 A1**
(43) Date of publication of application: **23.12.2009**
(21) Application number: 08158454.2
(22) Date of filing: 18.06.2008
(51) Int. Cl.: C07D 477/12, A61K 31/40, A61P 31/04

(54) **New trinem antibiotics and inhibitors of beta-lactamases**

(71) Applicant: LEK Pharmaceuticals d.d., 1526 Ljubljana (SI)
(72) Inventor: PLANTAN, Ivan, 1526 Ljubljana (SI); PREZELJ, Andrej, 1526 Ljubljana (SI); URLEB, Uros, 1526 Ljubljana (SI); MOHAR, Barbara, 1000 Ljubljana (SI); STEPHAN, Michel, 1000 Ljubljana (SI)
(74) Representative: Wichmann, Hendrik

(57) **Abstract**

The present invention relates to a compound of formula (I) 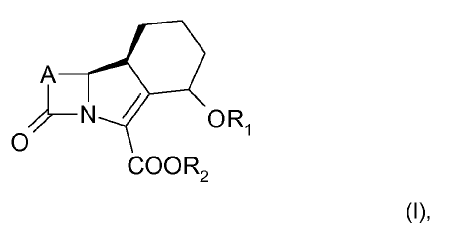 in particular compounds of formula (Ia), 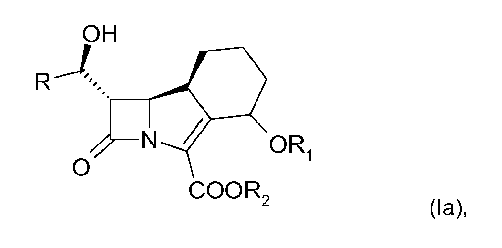 the use of a therapeutically effective amount of one or more compounds of formula (I) or (Ia) as a broad-spectrum antibiotic and the use of a pharmaceutical composition comprising said compounds for the treatment of bacterial infections in humans or animals.

## Description

### FIELD OF INVENTION

The present invention relates to new antimicrobial compounds of formula (I), in particular of formula (Ia) or (Ib) or pharmaceutically acceptable salts, esters or amides thereof. The present invention relates also to synthetic intermediates for their preparation. Another subject of the invention concerns pharmaceutical compositions comprising at least a compound of formula (I) and their use for the treatment of bacterial infections in humans or animals.

### BACKGROUND OF THE INVENTION

The dramatic worldwide increase in the number of bacterial strains acquiring resistance to the beta-lactam antibiotics has become one of the most important threats to modern health care. The dissemination of existing beta-lactamases and the evolution of new enzymes with extended substrate profiles, are the most common and often the most efficient mechanism of bacterial resistance to beta-lactam antibiotics. Currently the beta-lactamase super-family has more than 550 members, many of which differ only by a single amino acid. Based on amino-acid sequence similarities, beta-lactamases have been broadly grouped into four molecular classes, A, B, C and D. [Bush K; et al; Antimicrob. Agents Chemother. 1995, 39 (6): 1211-1233; Thomson KS; et al; Microbes and Infections 2000, 2: 1225-1235].

Without being limited to any particular theory or mechanism of action, it is believed that bacteria use several different mechanisms to escape from beta-lactam antibiotics [Li et al., Antimicrob Agents Chemother 1995, 39:1948-1953]. β-lactamases are endogenous bacterial enzymes that destroy β-lactam antibiotics and eliminate their efficacy. The bacterial beta-lactamase enzymes hydrolyze antibiotics of beta-lactam family, e.g. penicillins, cephalosporins, monobactams, carbapenems, to inactive products by hydrolyzing the beta-lactam bond. One counter-strategy is to co-administer inhibitor of beta-lactamases such as clavulanate, sulbactam, or tazobactam, that have been successfully used in combinations against bacteria producing the ubiquitous and prevalent TEM-1 and SHV-1 class A beta-lactamases. However, little or no activity against class C and B enzymes was observed. In addition, bacterial susceptibility to such combinations has recently been challenged by the spontaneous appearance of new beta-lactamases of the TEM family, which are resistant to the mechanism-based inactivators in the market. Any organism with an inducible AmpC beta-lactamase (class C) can segregate derepressed mutants, and any TEM, SHV or CTX-M producer can segregate ESBL (extended spectrum beta-lactamase) variants. [Livermore, DM. J. Antimicrob. Chemother. 1998, 41 (D), 25-41; Livermore, DM. Clinical Microbiology Reviews 1995, 8 (4), 557-584; Helfand MS; et al; Curr. Opin. Pharmacol. 2005, 5: 452-458]. Attempts to address the above mentioned problems through the development of inhibitor of beta-lactamases had only limited success in the past.

Alkylidene penems and 2-beta-substituted penam sulphones, oxapenems, cephalosporin-derived compounds, cyclic acyl phosphonates, and non-beta-lactam compounds are currently under investigation as potential inhibitors of beta-lactamases, but their clinical applications are not yet available [Buynak JD. Curr. Med. Chem. 2004, 11, 1951-1964; Bonnefoy A et al, J. Am. Chem. Soc. 2004, 54, 410-417; Weiss WJ et al; Antimicrob. Agents Chemother. 2004, 48, 4589-4596, Phillips OA at al; J. Antibiot. 1997, 50, 350-356; Jamieson CE et al; Antimicrob. Agents Chemother. 2003, 47, 1652-1657].

Several beta-lactam antibiotics that are stable to clinically relevant beta-lactamases have been designed by introducing bulky substituents that sterically hinder binding to the beta-lactamases. Sanfetrinem cilexetil (GV-118819) is an orally absorbed prodrug ester of sanfetrinem sodium (GV-104326), a highly potent broad-spectrum tricyclic beta-lactam antibiotic (trinem) which is active *in vitro* and *in vivo* against a wide range of Gram-positive, Gram-negative and anaerobic bacteria, except *Pseudomonas aeruginosa* and methicillin-resistant *Staphylococcus aureus.* Its activity was superior to several cefalosporins against *Escherichia coli*, *Klebsiella pneumoniae*, *Klebsiella oxytoca*, *Citrobacter diversus, Proteus mirabilis, Proteus vulgaris, Morganella morganii, Providencia rettgeri*, *Haemophilus* spp., and *Moraxella catarrhalis.* This compound is active against *Enterococcus faecalis*, *Enterococcus faecium*, *S. aureus*, streptococci, *Rhodococcus*-like species, and anaerobes. [SK Spangler, et al, Antimicrob. Agents Chemother. 1997, 41, 1, 148-155].

Therefore, one point of interest according to the present invention is the improvement of the stability of enzyme-inhibitor complexes through the design of efficient compounds with high acylation and low deacylation rates that are resistant to inactivation by beta-lactamases. Another subject of the present invention is to provide new pharmaceutical compositions that show a broad spectrum of potency against the most prevalent clinically relevant resistant strains.

It has been found that antibiotic activity of compounds of formula (I) and in particular of formula (Ia) provide a broad coverage against clinically important pathogens.

Moreover, we found that compounds of formula (I) are suitable inhibitors of beta-lactamases to combat emerging bacterial resistance in class A, class C and class D beta-lactamases.

### SUMMARY OF THE INVENTION

The present invention is directed towards a compound of formula (I) 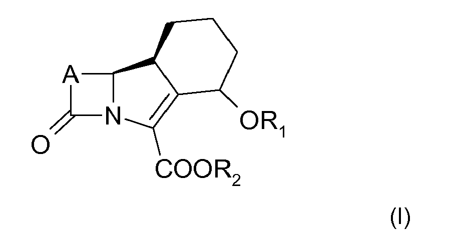 wherein A is defined as 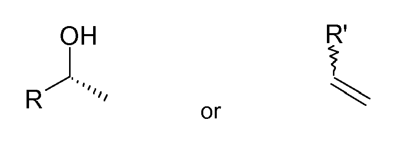 and wherein
R represents a C₁₋₂₀ fluorine- or chlorine-containing hydrocarbon moiety, straight, branched or cyclic, where the hydrocarbon moitey can be optionally interrupted or substituted by unsaturations;
R' represents a a C₁₋₂₀ fluorine- or chlorine-containing hydrocarbon moiety, straight, branched or cyclic, where the hydrocarbon moitey can be optionally interrupted or substituted by unsaturations;
R¹ represents a hydrogen atom, a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon);
R² represents: a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

In general formula (I), the group A is attached by the bonds marked as (x) and (y):

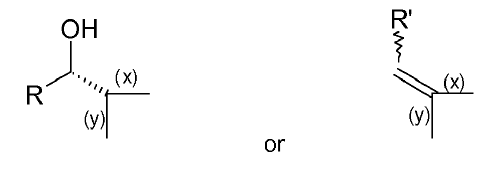

Thus, general formula (I) represents for example compounds of general formula (Ia) and (Ib).

The present invention also relates to a pharmaceutical composition at least comprising one compound of formula (I) as disclosed above.

According to a preferred embodiment, the present invention also provides a pharmaceutical composition as disclosed above, wherein the compound of formula (I) is a compound of the formula (Ia)

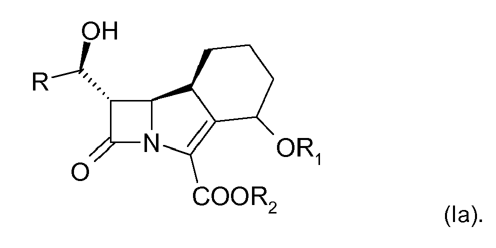

According to a further preferred embodiment, the present invention also provides a pharmaceutical composition as disclosed above, wherein the compound of formula (I) is a compound of the formula (Ib)

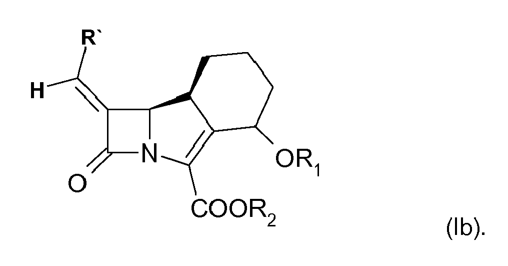

Furthermore, this invention relates to a process for the preparation of the compounds of the general formula (I), in particular of formula (Ia), and respective intermediates of the process.

Additionally, the present invention is directed to the use of compounds of formula (I) as defined above as a broad-spectrum antibiotic, in particular with additional beta-lactamase inhibitor activity against beta-lactamases of class A, C and D.

The present invention is also directed to the use of a compound of formula (I) or a pharmaceutical composition as disclosed above for the treatment of a bacterial infection in humans or animals.

Finally, the present invention is also directed to the use of a therapeutically effective amount of the compound or the pharmaceutical composition according to the present invention and at least one pharmaceutically acceptable excipient for the preparation of a medicament for treating a bacterial infection, preferably wherein said medicament is to be administered to a patient in need thereof.

Additionally, the present invention is directed to a method of treating a bacterial infection in humans or animals comprising administering to a patient in need of such treating a therapeutically effective amount of the compound or the pharmaceutical composition according to the present invention and at least one pharmaceutically acceptable excipient.

### DETAILED DESCRIPTION OF THE INVENTION

The present invention relates to a compound of formula (I) 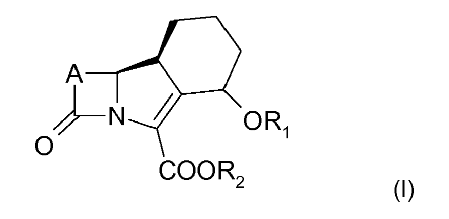 wherein
- A is defined as 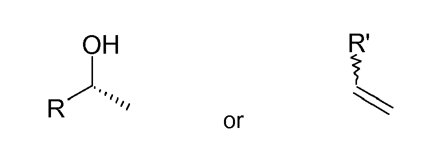 and wherein
- R represents a C₁₋₂₀ fluorine- or chlorine-containing hydrocarbon moiety, straight, branched or cyclic, where the hydrocarbon moitey can be optionally interrupted or substituted by unsaturations;
- R' represents a a C₁₋₂₀ fluorine- or chlorine-containing hydrocarbon moiety, straight, branched or cyclic, where the hydrocarbon moitey can be optionally interrupted or substituted by unsaturations;
- R¹ represents a hydrogen atom, a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon);
- R² represents: a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

According to the present invention, A is defined as

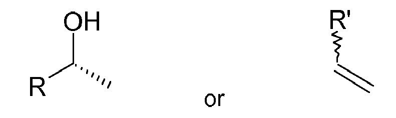

In general formula (I), the group A is attached by the bonds marked as (x) and (y):

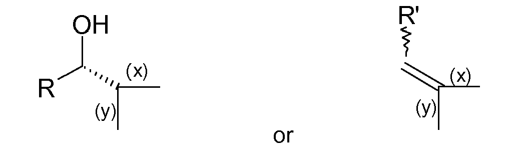

Therefore, the compound of formula (I) represents a molecule which has a C-C-single bond or a C-C-double bond, each resulting in several possible isomers which are all subject of the present invention.

Possible compounds of formula (I) are for example the following compounds of formula (Ia) to (Id):

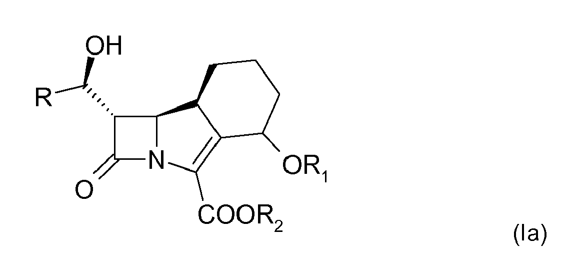

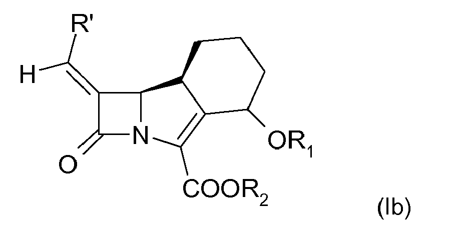

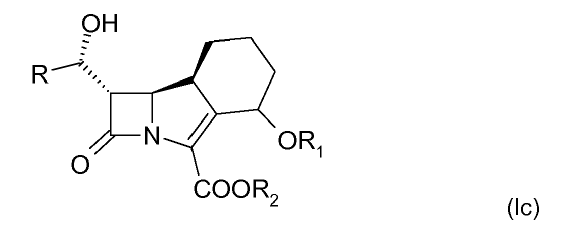

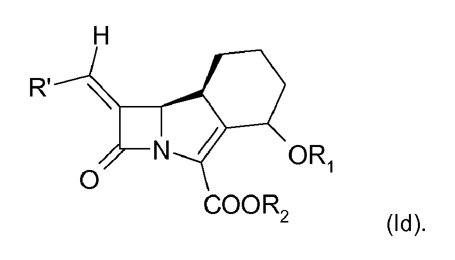

Preferably, the compound of formula (I) is a compound of formula (Ia) or (Ib):

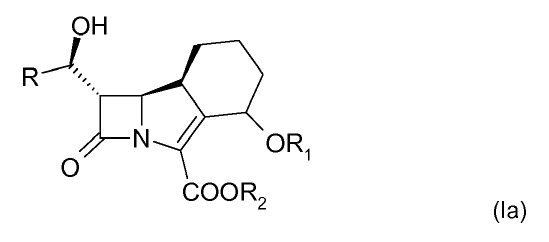

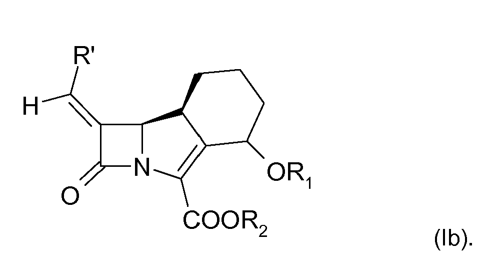

Particularly preferred are compounds of general formula (Ia') or (Ib'):

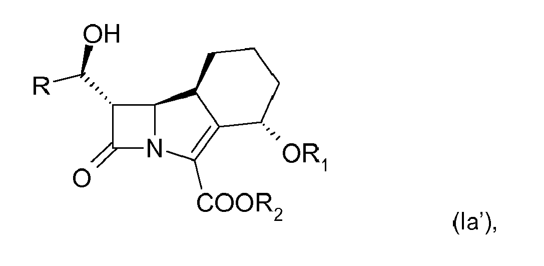

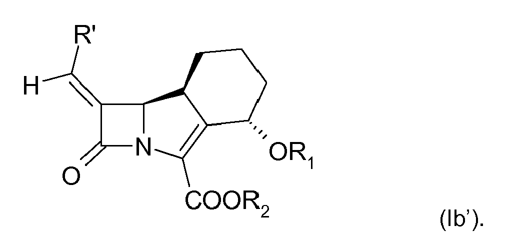

The structure of the molecule leads to stereoisomers which are within the scope of the present invention including mixtures thereof; a preferred embodiment of the present invention consists of diastereomerically enriched compounds with the predominant stereo and geometrical isomers represented by general formulae (Ia) or (Ib).

The present invention is also directed to a pharmaceutical composition at least comprising one compound as disclosed above.

According to a preferred embodiment, the present invention therefore also provides a pharmaceutical composition as disclosed above, wherein the compound of formula (I) is a compound of the formula (Ia)

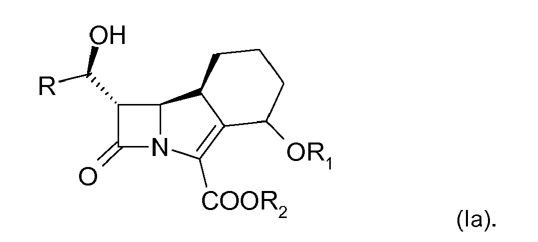

According to a further preferred embodiment, the present invention also provides a pharmaceutical composition as disclosed above, wherein the compound of formula (I) is a compound of the formula (Ib)

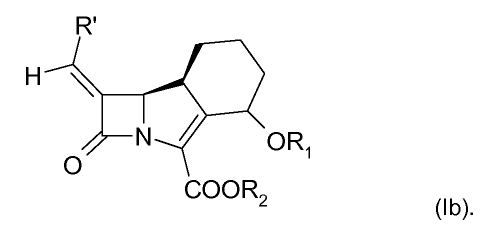

According to a further embodiment, the present invention also relates to a compound of formula (Ia) 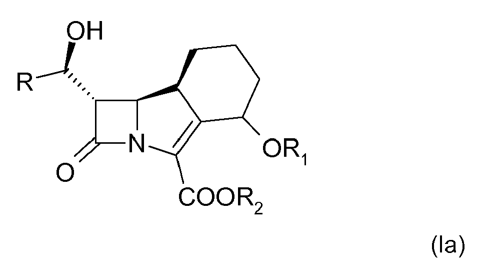 wherein
- R represents a C₁₋₂₀ fluorine- or chlorine-containing hydrocarbon moiety, straight, branched or cyclic, where the hydrocarbon moitey can be optionally interrupted or substituted by unsaturations;
- R' represents a C₁₋₂₀ fluorine- or chlorine-containing hydrocarbon moiety, straight, branched or cyclic, where the hydrocarbon moitey can be optionally interrupted or substituted by unsaturations;
- R¹ represents a hydrogen atom, a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon);
- R² represents: a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

In the context of the present invention, all reference to the compound of formula (I) also includes the preferred embodiments of formula (Ia) and (Ib) or (Ia') and (Ib') unless otherwise noted.

The residue R according to the present invention a C₁₋₂₀ fluorine- or chlorine-containing hydrocarbon moiety, straight, branched or cyclic, where the hydrocarbon moitey can be optionally interrupted or substituted by unsaturations.

Preferably, R represents a C₁₋₃ fluorine- or chlorine-containing hydrocarbon moiety such as fluoromethyl, difluoromethyl, trifluoromethyl, 2-chloroethyl, and more particularly fluoromethyl.

R may preferably represent CFH₂ group, a CF₂H group or a CF₃ group.

The residue R' may represent according to the present invention a C₁₋₂₀ fluorine- or chlorine-containing hydrocarbon moiety, straight, branched or cyclic, where the hydrocarbon moitey can be optionally interrupted or substituted by unsaturations.

Preferably, R' represents an alkyl residue with 1 to 5 carbon atoms such as methyl or ethyl, in particular methyl.

According to one embodiment of the present invention, R' preferably represents a mono-, di- or trisubstituted halo-alkyl chain with 1 to 3 carbon atoms such as fluoromethyl, difluoromethyl, trifluoromethyl, 2-chloroethyl, in particular fluoromethyl. R' may preferably represent CFH₂ group, a CF₂H group or a CF₃ group.

The residue R¹ may represent according to the present invention a hydrogen atom, a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

Preferably, R¹ represents a methyl, ethyl, n-propyl, isopropyl, n-butyl, s-butyl, isobutyl, tert-butyl, isoamyl, cyclopropyl, cyclohexyl, 1-cyclohexenyl, cycloheptyl, 2-tetrahydrofuranyl, vinyl, propenyl, allyl, propargyl, fluoromethyl, trifluoromethyl, 2-chloroethyl, hydroxymethyl, 2-hydroxyethyl, methoxymethyl, 2-methoxyethyl, mercaptomethyl, 2-methylmercaptoethyl, methanesulfonylmethyl, 2-aminoethyl, 2-methylaminoethyl, 2-dimethylaminoethyl, 2-(N-piperidino)ethyl, N-pyrrolidinomethyl, guanidinomethyl, iminomethylaminomethyl, 2-(dimethylaminomethyleneamino)ethyl, carbethoxymethyl, 2-cyanoethyl, acetyl, propionyl, isopropionyl, allylcarbonyl, pivaloyl, benzoyl, nitrobenzoyl, ethoxycarbonyl, t-butoxycarbonyl, allyloxycarbonyl, 2,2,2-trichloroethoxycarbonyl, 1,1,1-trichloro-2-methyl-2-propoxycarbonyl, benzyloxycarbonyl, nitrobenzyloxycarbonyl, methanesulfonyl, ethanesulfonyl, allylsulfonyl, nitrobenzylsulfonyl, trimethylsilyl, tert-butyldimethylsilyl; preferably R¹ represents a C₁₋₆ hydrocarbon moiety such as methyl or ethyl, an alkanoyl or aroyl, alkoxycarbonyl and more particularly a methyl.

When the group OR¹ is a protected hydroxyl group this is conveniently an ether or an acyloxy group. Examples of particularly suitable ethers include those in which R¹ is a hydrocarbylsilyl group such as trialkylsilyl, e.g. trimethylsilyl or t-butyldimethylsilyl. When the group OR¹ represents an acyloxy group then examples of suitable groups R¹ includes alkanoyl e.g. acetyl, pivaloyl; alkenoyl e.g. allylcarbonyl; aroyl e.g. p-nitrobenzoyl; alkoxycarbonyl e.g. t-butoxycarbonyl; haloalkoxycarbonyl e.g. 2,2,2-trichloroethoxycarbonyl, or 1,1,1-trichloro-2-methyl-2-propoxycarbonyl; aralkyloxycarbonyl e.g. benzyloxycarbonyl or P-nitrobenzyloxycarbonyl; or alkenyloxycarbonyl e.g. allyloxycarbonyl. In particular, R¹ is trimethylsilyl.

According to the present invention, the residue R² may represent a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

For example, R² represents a methyl, ethyl, tert-butyl, allyl, phenetyl, 2,2,2-trichloroethyl, trimethylsilyl, tert-butyldimethylsilyl, 2-trimethylsilylethyl, benzyl, methoxybenzyl, nitrobenzyl, bis(methoxyphenyl)methyl, 3,4-dimethoxybenzyl, benzhydryl, trityl, 2-(N-morpholino)ethyl, methoxymethyl, 2-methoxyethyl, mercaptomethyl, 2-methylmercaptoethyl, a degradable esters (as to constitute a prodrug) forming a group as for example: acetoxyethyl, pivaloyloxymethyl, pivaloyloxyethyl, cyclohexoyloxyethyl, benzoyloxyethyl, [(1-methoxy-1-methyl)ethylcarbonyloxy]methyl, [(1-methoxy-1-methyl)ethylcarbonyloxy]ethyl, (isopropoxycarbonyloxy)ethyl, (cyclohexylmethyloxycarbonyloxy)methyl, (cyclohexyloxycarbonyloxy)methyl, (cyclohexyloxycarbonyloxy)ethyl, (4-ethylcyclohexyloxycarbonyloxy)ethyl, (phenoxycarbonyloxy)ethyl, (2-oxo-5-methyl-1,3-dioxolane-4-yl)methyl.

Preferably R² represents a hydrogen, a metal cation (in particular Na⁺, K⁺, Ca²⁺), ammonium ion, a methyl, ethyl, allyl, 2-(N-morpholino)ethyl, 1-acetoxyethyl, pivaloyloxymethyl, 1-(pivaloyloxy)ethyl, 1-(cyclohexoyloxy)ethyl, 1-(benzoyloxy)ethyl, [(1-methoxy-1-methyl)ethylcarbonyloxy]methyl, 1-[(1-methoxy-1-methyl)ethylcarbonyloxy]ethyl, 1-(isopropoxycarbonyloxy)ethyl, (cyclohexylmethyloxycarbonyloxy)methyl, (cyclohexyloxycarbonyloxy)methyl, 1-(cyclohexyloxycarbonyloxy)ethyl, 1-(4-ethylcyclohexyloxycarbonyloxy)ethyl, 1-(phenoxycarbonyloxy)ethyl, (2-oxo-5-methyl-1,3-dioxolane-4-yl)methyl.

According to a preferred embodiment, the compound according to formula (I), in particular the compound of formula (Ia) or (Ib) or (Ia') or (Ib') is an acid, a salt or an ester.

According to a further embodiment, the present invention therefore provides a compound as disclosed above, wherein in formula (I), formula (Ia) or formula (Ib), R represents a CFH₂ group, a CF₂H group or a CF₃ group, and/or R¹ represents a methyl group, and the residue R² represents an optionally functionalised hydrocarbon.

Furthermore, the present invention is also directed to a compound of the general formula (Ia) 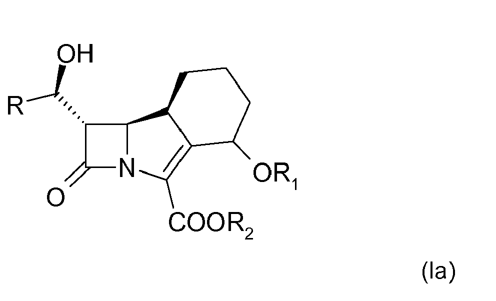 wherein
R represents a CFH₂ group, a CF₂H group or a CF₃ group,
R¹ represents a methyl group, and
R² represents: a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

According to especially preferred embodiments, the present invention is directed to compound of formula (Ia), wherein R² represents a hydrogen atom. Furthermore, R preferably represents a CH₂F group.

According to a further embodiment, the present invention is directed to compound of formula (Ia) wherein
- R represents a fluoromethyl group,
- R¹ represents a methyl group,
- R² represents a hydrogen atom or a metal cation as Li⁺, Na⁺ or K⁺.

Furthermore, the present invention provides a compound of formula (Ia) as disclosed above, wherein
- R represents a CH₂F group, and
- R² represents a hydrogen atom.

These compounds of formula (I), in particular of formula (Ia) can be prepared by any suitable method known to the person skilled in the art.

The present invention also relates to a process for the preparation of the compounds of the general formula (I), in particular the compound of formula (Ia) or (Ib) or of formula (Ia') or (Ib'). 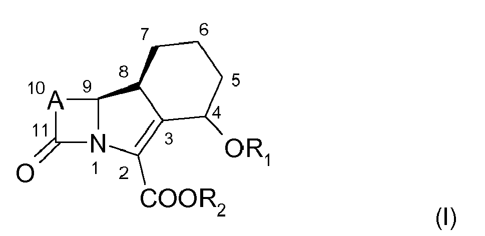 wherein A represents 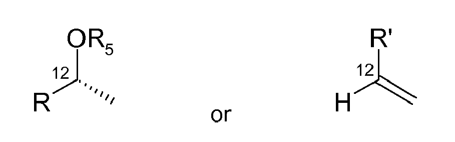 wherein R, R¹, and R² are as defined above. R⁵ represents a hydrogen atom or a hydroxyl protecting group; compounds wherein R⁵ represents a hydroxyl protecting group.

Suitable hydroxyl protecting groups R⁵ include those which may be removed by hydrolysis under buffered conditions or under non-aqueous conditions. When the group OR⁵ is a protected hydroxyl group this is conveniently an ether or an acyloxy group. Examples of particularly suitable ethers include those in which R⁵ is a hydrocarbylsilyl group such as trialkylsilyl, e.g. trimethylsilyl or t-butyldimethylsilyl. When the group OR₁ represents an acyloxy group then examples of suitable groups R⁵ includes alkanoyl e.g. acetyl, pivaloyl; alkenoyl e.g. allylcarbonyl; aroyl e.g. p-nitrobenzoyl; alkoxycarbonyl e.g. t-butoxycarbonyl; haloalkoxycarbonyl e.g. 2,2,2-trichloroethoxycarbonyl, or 1,1,1-trichloro-2-methyl-2-propoxycarbonyl; aralkyloxycarbonyl e.g. benzyloxycarbonyl or P-nitrobenzyloxycarbonyl; or alkenyloxycarbonyl e.g. allyloxycarbonyl.

A particularly convenient protecting group R⁵ is trimethylsilyl.

The general formula of compound (I) as drawn can include at least 4 to 5 stereogenic centers. This represents at least 16 to 32 posible stereoisomers and mixtures thereof. Particulary useful compounds represent compounds with configuration S on carbon atom 4, S on carobon atom 8, R on carbon 9, S on carbon 10, and R on carbon 12 if possible or E configuration of exocyclic double bond. The assignment of the R or S configuration at those centers have been made according to the rules of Cahn. Ingold and Prelog, Experientia 156, 12, 81.

Specific preferred compounds include (4S,8S,9R,10S,12R)-4-methoxy-10-(-2-fluoro-1-hydroxyethyl)-11-oxo-1-azatricyclo[7.2.0.0^{3,8}] undec-2-ene-2-carboxylic acid and salts therof e.g. sodium, lithium or potassium salt.

According to a preferred embodiment of the present invention, compounds of the formula (I) may be prepared by multistep synthesis from commercially available starting compounds. Therefore, the present invention is also directed to a process for preparing a compound of general formula (I) as defined above, comprising the steps:
(a) asymmetric reduction of a compound of general formula (II) to obtain a compound of general formula (III): 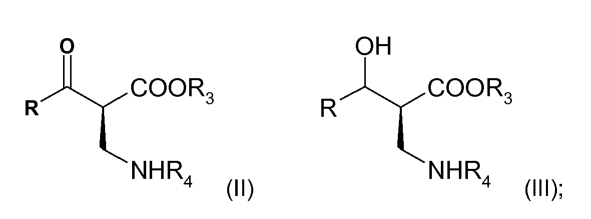
(b) cyclisation reaction to obtain a compound of general formula (IV): 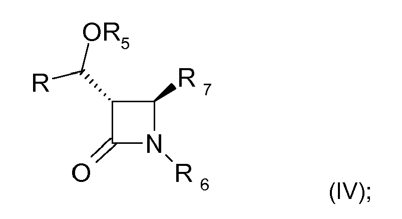
(c) preparation of a compound of general formula (V): 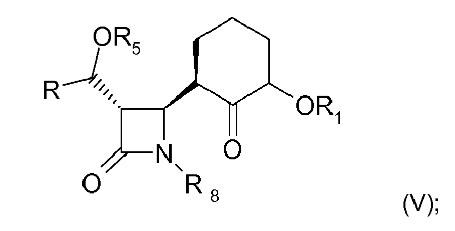
(d) cyclisation reaction to obtain a compound of general formula (VI): 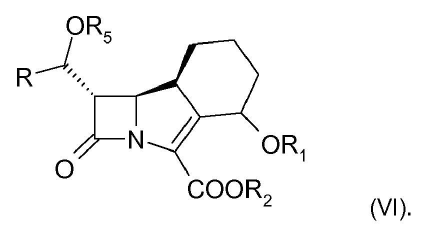 In the general formulas given above, residues R¹ and R² are defined as above, R³ represents a carboxyl protecting group, preferably an ethyl radical, and R⁴ represents an amino protecting group, preferably a benzoyl radical, R⁵ represents a hydrogen atom or a hydroxyl protecting group.

The process according to the present invention comprises steps (a) to (d). The process can comprise further steps, in particular protection group modifications. Suitable methods for individual substituents as described for example in PROTECTIVE GROUPS in ORGANIC CHEMISTRY,T. W. Green, P.G. Wuts; John Wiley & Sons 1999).

The process according to the present invention preferably starts from a compound of general formula (II) which can be prepared by reacting a commercially available betaketo ester with a base and subsequent alkylation with a halomethylamine derivative. Sutiable bases are alkali and earth alkali metals, hydroxides, hydrides, carbonates and amides.

According to step (a), a compound of formula (III) is prepared: 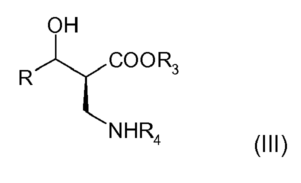 wherein R represents a residue as defined above, R³ represents a carboxy protecting group, preferably ethyl, and R⁴ represents an amino preotecting group, preferably benzoic acid.

The compound of formula (III) is prepared by asymmetric reduction of the compound of formula (II). This can be achieved in stereo selective manner. Suitable reaction conditions are known to the skilled person and various known techniques can be employed.

According to step (b), a compound of formula (IV) is prepared by cyclisation: 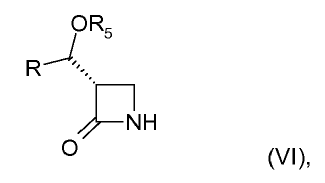 wherein R and R⁵ represent residues as defined above.

The compound of formula (IV) can be prepared by cyclisation. This is done preferably by activating the carboxylic group with a variety of reagents, preferably 2,2'-dipyridyl disulfide and triphenyl phospine in a suitable solvent such as acetonitrile (CAN) or dimethylsulfoxide (DMSO) at elevated temperatures (PROTECTIVE GROUPS in ORGANIC CHEMISTRY,T. W. Green, P.G. Wuts; John Wiley & Sons 1999). Suitable reaction conditions are known to the skilled person and various known techniques can be employed.

According to step (c), a compound of formula (V) is prepared: 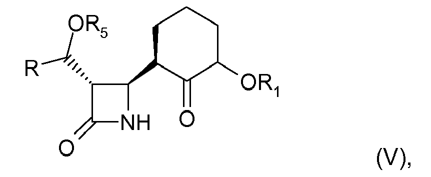 wherein R, R¹, and R⁵ represent residues as described above, using a compound of formula (Va): 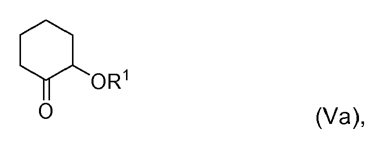 wherein R¹ is a residue as defined above. Alternatively, the compound of formula (V) can also be prepared using compound of formula (Vb): 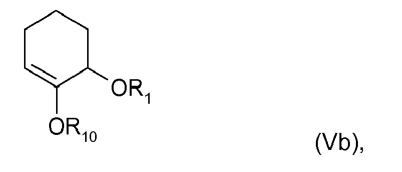 wherein R¹ is a residue as described above, R¹⁰ is an easily removable enol-protecting group.

The reaction can be carried out by reacting the starting compound with or without a strong base and with or without a Lewis acid. Suitable reaction conditions are known to the skilled person and various known techniques can be employed.

According to step (d), a compound of formula (VI) is prepared: 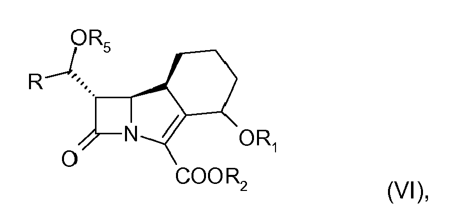 wherein R, R¹, R², and R⁵ represent residues as described above.

The compound of formula (VI) is prepared by cyclization. Suitable reaction conditions are known to the skilled person and various known techniques can be employed.

The process according to the present invention is not limited to the above-described reaction route and can comprise further steps. The process can also comprise a further elimination step after step (d) to obtain a compound of general formula (Ib).

Furthermore, this invention relates to synthetic intermediates (III), (IV), (V), and (VI) for the preparation of the compounds of the general formula (I), and in particular of formulae (Ia) or (Ib):

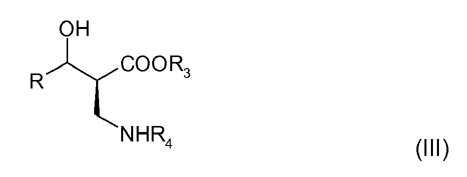

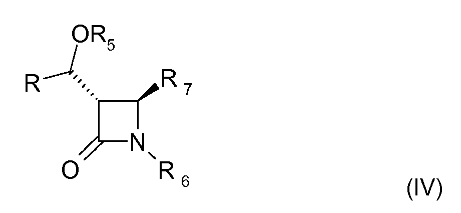

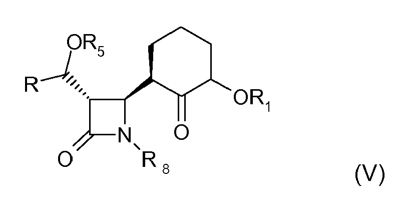

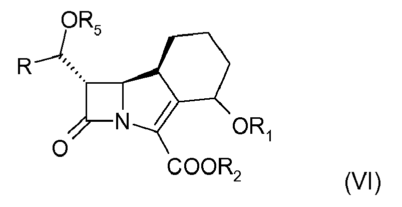

In intermediate (III),
- R represents as described in formula (I),
- R³ represents a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine), a C₁₋₂₀ hydrocarbon moiety, straight, branched or cyclic; the hydrocarbon moitey can be optionally interrupted or substituted by unsaturations, C₅₋₆ arenes in such a way that R³ forms for example: a methyl, ethyl, propyl, allyl, benzyl, methoxybenzyl, nitrobenzyl, and more particularly R³ is a hydrogen atom, methyl, ethyl, propyl,
- R⁴ represents a hydrogen atom, a C₁₋₁₀ alkanoyl or aroyl (such as acetyl, propionyl, isopropionyl, allylcarbonyl, pivaloyl, benzoyl, nitrobenzoyl), alkoxycarbonyl or arylmethoxycarbonyl (such as ethoxycarbonyl, t-butoxycarbonyl, allyloxycarbonyl, 2,2,2-trichloroethoxycarbonyl, 1,1,1-trichloro-2-methyl-2-propoxycarbonyl, benzyloxycarbonyl, nitrobenzyloxycarbonyl), and more particularly R⁴ is a hydrogen atom, acetyl, benzoyl.

In intermediate (IV),
- R represents as described in formula (I),
- R⁵ represents a hydrogen atom or a hydroxy protecting group, for example, a silyl group (such as trimethylsilyl, t-butyldimethylsilyl, triisopropylsilyl), a C₁₋₁₀ acyl or aroyl (such as acetyl, pivaloyl, allylcarbonyl, benzoyl, nitrobenzoyl), and more particularly R⁵ is a hydrogen atom or trimethylsilyl,
- R⁶ represents a hydrogen atom or an amide protecting group such as trimethylsilyl, t-butyldimethylsilyl, triisopropylsilyl, acetyl, pivaloyl, allylcarbonyl, benzoyl, and more preferably a hydrogen atom or trimethylsilyl,
- R⁷ represents a hydrogen atom, a leaving group such as acetoxy.

In intermediate (V),
- R represents as described in formula (I),
- R¹ represents as described in formula (I),
- R⁵ represents as described in formula (III),
- R³ represents a hydrogen atom, an oxalyl group such as allyloxalyl, benzyloxalyl.

In intermediate (VI),
- R represents as described in formula (I),
- R¹ represents as described in formula (I),
- R² represents as described in formula (I),
- R⁵ represents as described in formula (III).

Compounds of formula (I) may be prepared through intermediates (II), (III), (IV), (V) and (VI) as disclosed above. An example of such synthesis is depicted in the following scheme, which represents the preparation given in the examples.

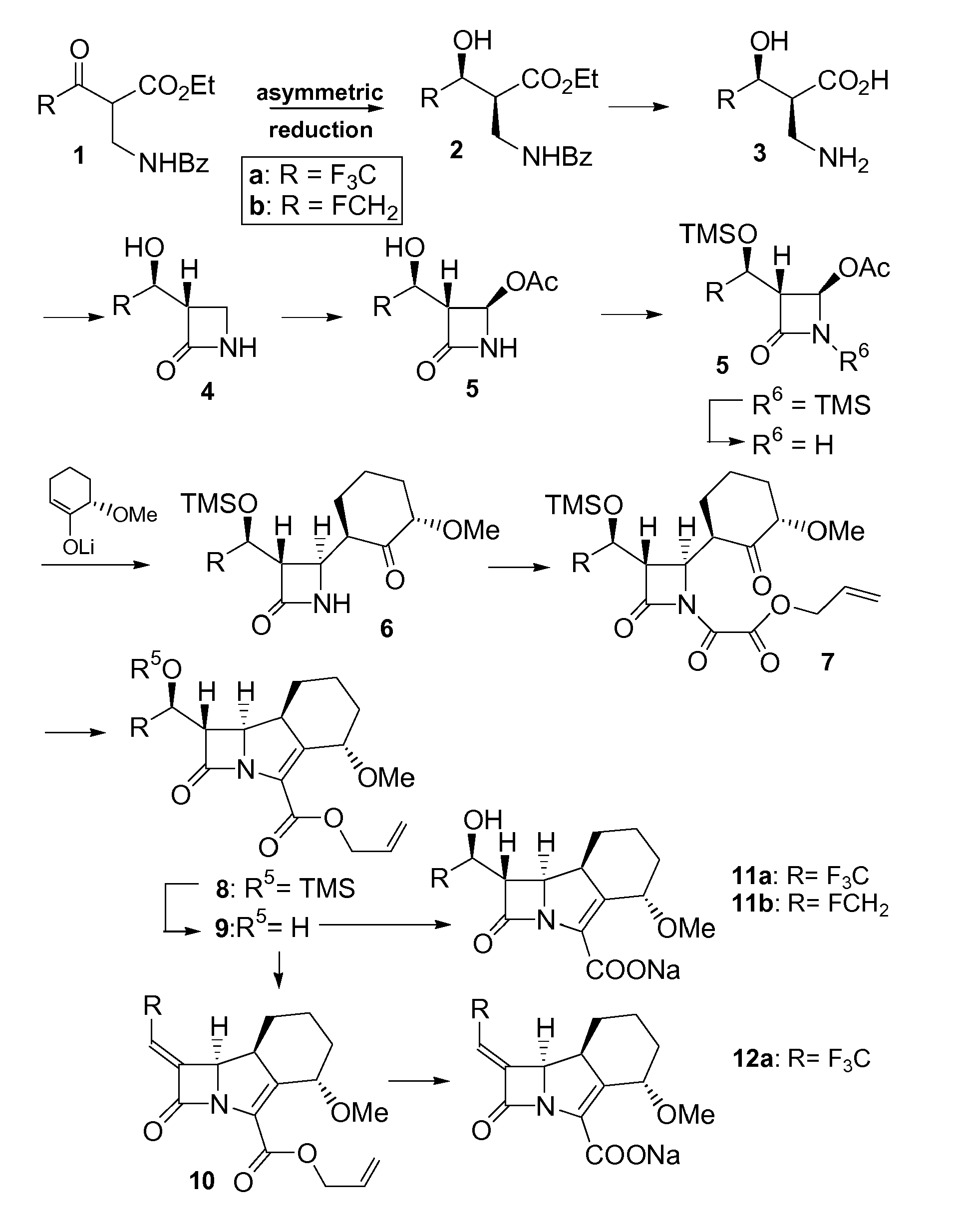

The compounds of formula (I), in particular of formula (Ia) and (Ib) as disclosed above react as an antibiotic with inherent activity as inhibitor of beta-lactamases.

Antibiotics of formula (I) in particular of formula (Ia) and (Ib) are very potent inhibitors of beta-lactamases that inhibit enzymatic activity of beta-lactamases *in vitro* and enhance the potency of antibiotic agents in bacterial cell culture and are useful for the treatment of bacterial infections in humans and animals.

Various pharmaceutically acceptable salts, ether derivatives, ester derivatives, acid derivatives, and aqueous solubility altering derivatives of the compound of formula (I), in particular the compound of formula (Ia) or (Ib), also are encompassed by the present invention. The present invention further includes all individual enantiomers, diastereomers, racemates, and other isomer of the compound. The invention also includes all polymorphs and solvates, such as hydrates and those formed with organic solvents, of this compound. Such isomers, polymorphs, and solvates may be prepared by methods known in the art, such as by regiospecific and/or enantioselective synthesis and resolution, based on the disclosure provided herein.

Isomeric mixtures may be separated as appropriate, e.g. according, e.g. analogously, to a method as conventional, to obtain pure isomers. The present invention includes a compound of the present invention in any isomeric form and in any isomeric mixture. The present invention also includes tautomers of a compound of the present invention, where tautomers can exist.

Suitable salts of the compound formula (I), in particular the compound of formula (Ia) or (Ib), include, but are not limited to, acid addition salts, such as those made with hydrochloric, hydrobromic, hydroiodic, perchloric, sulfuric, nitric, phosphoric, acetic, propionic, glycolic, lactic pyruvic, malonic, succinic, maleic, fumaric, malic, tartaric, citric, benzoic, carbonic cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid; salts made with saccharin; alkali metal salts, such as sodium and potassium salts; alkaline earth metal salts, such as calcium and magnesium salts; and salts formed with organic or inorganic ligands, such as quaternary ammonium salts.

Prodrugs and active metabolites of compounds disclosed herein are also within the scope of the invention. A prodrug and an active metabolite are terms well-known to the person skilled in the art.

According to a preferred embodiment, the present invention also relates to pharmaceutical compositions comprising at least a compound defined by formula (I), in particular by formula (Ia) or (Ib).

Furterhmore, the present invention also relates to the use of a therapeutically effective amount of one or more compounds of formula (I) as an antibiotic.

The term "antibiotic" as used herein describes a compound or composition which decreases the viability of a microorganism, or which inhibits the growth or reproduction of a microorganism. "Inhibits the growth or reproduction" means increasing the generation cycle time by at least 2-fold, preferably at least 10-fold, more preferably at least 100-fold, and most preferably indefinitely, as in total cell death. An antibiotic is further intended to include an antimicrobial, bacteriostatic, or bactericidal agent. Non-limiting examples of antibiotics useful according to the present invention include penicillins, cephalosporins, aminoglycosides, sulfonamides, macrolides, tetracyclins, lincosides, quinolones, chloramphenicol, vancomycin, metronidazole, rifampin, isoniazid, spectin-omycin, trimethoprim, sulfamethoxazole, and others.

The term "beta-lactam antibiotic" as used herein designates compounds with antibiotic properties containing a beta-lactam functionality.

The pharmaceutical composition of the present invention may also comprise further compounds such as conventional non-toxic pharmaceutically acceptable carrier, adjuvants or vehicles. Preferably, the compounds used in the pharmaceutical compositions of the invention are formulated in pharmaceutical compositions by combining the compounds with any conventional non-toxic pharmaceutically acceptable carrier, adjuvants or vehicles.

Thus, the present invention is also directed to a pharmaceutical composition comprising an antibiotic with inherent beta-lactamase inhibitory activity of formula (I), in particular of formula (Ia) or (Ib), and a pharmaceutically acceptable carrier.

As used herein, the term "pharmaceutically acceptable" means a non-toxic material that does not interfere with the potency of the biological activity of the active ingredient(s). The term "physiologically acceptable" refers to a non-toxic material that is compatible with a biological system such as a cell, cell culture, tissue, or organism.

The term "pharmaceutically acceptable carrier" refers to a non-toxic carrier that may be administered to a patient, together with a compound of this invention in combination with antibiotics, preferably beta-lactam antibiotics, and which does not destroy the pharmacological activity thereof.

In general, all carriers known by a respective person skilled in the art are suitable for their use within the present pharmaceutical composition. Normally, the characteristics of the preferred carrier depend on the route of administration of the respective pharmaceutical composition.

Solid carriers which are usable according to the present invention are for example finely divided solids such as talc, clay, microcrystalline cellulose, silica, alumina and the like. Useful liquid carriers include water, alcohols or glycols or water- alcohol/glycol blends, in which the present compounds can be dissolved or dispersed at effective levels, optionally with the aid of non-toxic surfactants. Adjuvants such as fragrances and additional antimicrobial agents can also be added to optimize the properties for a respective use. The resultant liquid pharmaceutical compositions can be applied from an absorbent pad, used to impregnate bandages and other dressings, or sprayed onto the affected area using pump-type or aerosol sprayers.

For topical administration, it will generally be desirable to administer the present compounds to the skin as compositions or formulations, in combination with a dermatologically acceptable carrier, which may be a solid or a liquid. Topical applications may be formulated in carriers such as hydrophobic or hydrophilic bases to form ointments, creams, lotions, in aqueous, oleaginous or alcoholic liquids to form paints or in dry diluents to form powders. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user. Cream or ointment formulations which may be used for the drug are conventional formulations well known in the art.

The pharmaceutical composition of the present invention may also comprise a pharmaceutically acceptable excipient. Therefore, the present invention is also directed to a pharmaceutical composition as disclosed above, wherein the pharmaceutical composition additionally comprises a pharmaceutically acceptable excipient.

In general all excipients known by a person skilled in the art are suitable within the present invention. Examples of such excipients are calcium carbonate, kaolin, sodium hydrogen carbonate, lactose, D-mannitol, starches, crystalline cellulose, talc, granulated sugar, porous substances, etc.

The compounds of formula (I) of the invention may be used as bulk itself but usually be formulated into pharmaceutical preparations together with a suitable amount of "carrier for pharmaceutical preparation" according to ordinary methods.

Thus, compositions and methods according to the invention may also contain additionally diluents, fillers, salts, buffers, stabilizers, solubilizers, and other materials well known in the art.

Further on, "carriers for pharmaceutical preparation" comprises, for example, excipients as defined above, binders, e.g., dextrin, gums, α-starch, gelatin, hydroxypropyl cellulose, hydroxypropylmethyl cellulose, pullulan, etc., thickening agents, e.g., natural gums, cellulose derivatives, acrylic acid derivatives, etc., disintegrators, e.g., carboxymethyl cellulose, croscarmellose sodium, crospovidone, low-substitution hydroxypropyl cellulose, partial α-starch, etc., solvents, e.g., water for injections, alcohol, propylene glycol, macrogol, sesame oil, corn oil, etc., dispersants, e.g., Tween 80, HCO60, polyethylene glycol, carboxymethyl cellulose, sodium alginate, etc., solubilizers, e.g., polyethylene glycol, propylene glycol, D-mannitol, benzyl benzoate, ethanol, trisaminomethane, triethanolamine, sodium carbonate, sodium citrate, etc., suspending agents, e.g., stearyl triethanolamine, sodium lauryl sulfate, benzalkonium chloride, polyvinyl alcohol, polyvinylpyrrolidone, hydroxyethyl cellulose, etc., pain-reducing agents, e.g., benzyl alcohol, etc., isotonizing agents, e.g., sodium chloride, glycerin, etc., buffers, e.g., phosphates, acetates, carbonates, citrates, etc., lubricants, e.g., magnesium stearate, calcium stearate, talc, starch, sodium benzoate, etc., colorants, e.g., tar pigments, caramel, iron sesquioxide, titanium oxide, riboflavins, etc., tasting agent, e.g., sweeteners, flavors, etc., stabilizers, e.g., sodium sulfite, ascorbic acid, etc., preservatives, e.g., parabens, sorbic acid, etc., and the like.

Pharmaceutical compositions according to the present invention may also comprise other active factors and/or agents which enhance the inhibition of beta-lactamases and/or DD-peptidases.

The respective pharmaceutical compositions are effective against bacteria which do not produce beta-lactamases, but also especially effective against bacteria which produce significant amounts of beta-lactamases. Thus, pharmaceutical compositions according to the present invention are generally useful for controlling bacterial infections levels *in vivo* and for treating diseases or reducing the advancement or severity of effects, which are mediated by bacteria.

Suitable subjects for the administration of the formulation of the present invention include mammals, primates, man, and other animals. Typically the animal subject is a mammal, generally a domesticated farm mammal, e.g. horse, pig, cow, sheep, goat etc., or a companion animal, e.g. cat, dog etc.. *In vitro* antibacterial activity is predictive of *in vivo* activity when the compositions are administered to a mammal infected with a susceptible bacterial organism.

### Route of administration

Preferred methods of administration of the pharmaceutical compositions described above include oral and parenteral, e.g., i.v. infusion, i.v. bolus and i.m. injection formulated so that a unit dosage comprises a therapeutically effective amount of each active component or some submultiples thereof.

The compounds may be employed in powder or crystalline form, in liquid solution, or in suspension. Theses compounds may be formulated by any method well known in the art and may be prepared for administration by any route, including, without limitation, parenteral, oral, sublingual, by inhalation spray, transdermal, topical, intranasal, intratracheal, intrarectal via ophthalmic solution or ointment, rectally, nasally, buccally, vaginally or via implanted reservoir. The term parenteral as used herein includes subcutaneous, intracutaneous, intravenous, intramuscular, intra-articular, intrasynovial, intrasternal, intrathecal, intralesional and intracranial injection or infusion techniques.

In case the compound of formula (I) is an acid, the pharmaceutical composition according to the present invention is preferably administered parenteral, in particular intravenous. In case the compound of formula (I) is an ester, the pharmaceutical composition according to the present invention is preferably administered orally.

Pharmaceutical compositions for injection, a preferred route of delivery according to the present invention, may be prepared in unit dosage form in ampules, or in multidose containers. The composition will generally be sterile and pyrogen-free, when intended for delivery by injection into the subject. The injectable compositions may take such forms as suspensions, solutions, or emulsions in oily or aqueous vehicles, and may contain various formulating agents. Alternatively, the active ingredient may be in powder (lyophilized or non-lyophilized) form for reconstitution at the time of delivery with a suitable vehicle, such as sterile water.

Carriers suitable for an injectable pharmaceutical composition according to the present invention are typically comprised sterile water, saline or another injectable liquid, e.g., peanut oil for intramuscular injections. Also, various buffering agents, preservatives and the like can be included. The pharmaceutical composition according to the present invention may also be administered parenterally in a sterile medium. Depending on the vehicle and concentration used, the drug can either be suspended or dissolved in the vehicle. Advantageously, adjuvants such as local anaesthetic, preservative and buffering agents can be dissolved in the vehicle. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. It is also preferred to include isotonic agents, for example, sugars, buffers or sodium chloride. Prolonged absorption of the injectable compositions can be brought about by the use in the compositions of agents delaying absorption, for example, aluminium monostearate and gelatine. Intra-venous infusion is another possible route of administration for the compounds used according to the present invention.

Orally administrable pharmaceutical compositions according to the present invention may be in the form of tablets, capsules, powders, granules, lozenges, liquid or gel preparations, such as oral, topical, or sterile parenteral solutions or suspensions. The oral compositions may utilize carriers such as conventional formulating agents, and may include sustained release properties as well as rapid delivery forms. Such compositions and preparations should contain at least 0.1% of active compounds. The percentage of the compositions and preparations may, of course, be varied and may conveniently be between about 2 to about 60% of the weight of a given unit dosage form. The amount of active compound in such therapeutically useful compositions is such that an effective dosage level will be obtained.

Tablets and capsules for oral administration may be in unit dose presentation form, and may also contain conventional excipients such as binding agents, for example syrup, acacia, gelatine, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrates for example potato starch, or acceptable wetting agents such as sodium lauryl sulphate. The tablets may be coated according to methods well known to a person skilled in the art. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatine hydrogenated edible fats; emulsifying agents, for example lecithin, sorbitan monooleate, or acacia; non-aqueous vehicles which may include edible oils, for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

Pharmaceutical compositions according to the present invention may also be prepared in suitable forms for absorption through the mucous membranes of the nose and throat or bronchial tissues and may conveniently take the form of powder or liquid sprays or inhalants, lozenges, throat paints, etc. For medication of the eyes or ears, the preparations may be presented as individual capsules, in liquid or semi-solid form, or may be used as drops, etc.

For veterinary medicine, the composition may, for example, be formulated as an intramammary preparation in either long acting or quick-release bases.

### Use of the compound of formula (I) as an antibiotic with inherent beta-lactamase inhibitory activity

The invention provides novel antibiotic with inherent beta-lactamase inhibitory activity of formula (I), in particular of formula (Ia) or (Ib), described above as well as a pharmaceutical composition comprising a compound of the general formula (I), in particular of formula (Ia) or (Ib).

Therefore antibiotic with inherent beta-lactamase inhibitory activity of formula (I), in particular of formula (Ia) or (Ib), can extend action of the beta-lactam antibiotic in the combination to strains producing inhibitor-sensitive enzymes and additionaly improve its antibacterial spectrum.

The present invention relates to the use of compounds of formula (I), in particular of formula (Ia) or (Ib), as defined above as antibiotic with inherent beta-lactamase inhibitory activity.

Therefore, the present invention also provides a therapeutically effective amount of one or more compounds of formula (I) as defined above or a compound of formula (Ia) as defined above for use as an antibiotic, in particular wherein the antibiotic is a broad-spectrum antibiotic with beta-lactamase inhibitor activity, in particular against beta-lactamases of class A, C and D.

Spectrum gain due to antibiotic activity of the inhibitor of beta-lactamases of formula (I), in particular of formula (Ia) or (Ib), is expected to be superior to commercially available inhibitors of beta-lactamases, such as clavulanic acid, sulbactam and tazobactam which have no antibiotic activity per se.

Compounds of formula (I), in particular of formula (Ia) or (Ib), are especially suitable as antibiotics and inhibitors of beta-lactamases for therapeutic applications. They are also useful as pharmacological tools for *in vitro* or *in vivo* studies to investigate the mechanisms of antibiotic resistance, to help identify other therapeutic antibiotic agents or inhibitors of beta-lactamases, to identify which beta-lactamases are being expressed by a given microorganism, or to selectively inhibit one or more beta-lactamases in a microorganism.

Thus, the present invention also relates to the use of a therapeutically effective amount of antibiotic with inherent beta-lactamase inhibitory activity of formula (I), in particular of formula (Ia) or (Ib), as defined above.

According to a preferred embodiment the inhibitor of beta-lactamases is a broad spectrum inhibitor of class A, C and D beta-lactamases. It effectively inhibits most of the clinically relevant and prevalent TEM- and SHV-type enzymes (class A), AmpC (class C) and OXA-type enzymes (class D).

Thus, the present invention is also directed to the use of a therapeutically effective amount of one or more compounds of formula (I), in particular of formula (Ia) or (Ib), as defined above as a broad-spectrum beta-lactamase inhibitor, in particular wherein the beta-lactamase inhibitor is a beta-lactamase inhibitor of class A, C and D.

According to a further embodiment, the present invention also provides a therapeutically effective amount of one or more compounds of formula (I) or a compound of formula (Ia) as disclosed above for use as an antibiotic.

The pharmaceutical composition according to the present invention also provides the use of a therapeutically effective amount of one or more compounds of formula (I), in particular of formula (Ia) or (Ib), as defined above as an antibiotic.

Additionally, according to another embodiment the present invention comprising a broad spectrum inhibitor of beta-lactamases is clearly superior to current therapeutic options.

Therefore, the present invention also provides a therapeutically effective amount of one or more compounds of formula (I) as defined above or a compound of formula (Ia) as defined above for use as a broad-spectrum beta-lactamase inhibitor as described above, wherein the beta-lactamase inhibitor is a beta-lactamase inhibitor of class A, C and D.

### Inhibition of Bacterial Growth

In a further aspect, the present invention provides methods for inhibiting bacterial growth, such methods comprising administering a compound of formula (I) according to the present invention or a pharmaceutical composition according to the present invention comprising an antibiotic with inherent beta-lactamase inhibitory activity of formula (I), in particular of formula (Ia) or (Ib), to a bacterial cell culture, or to a bacterially infected cell culture, tissue, or organism.

It is known that the response to a given compound may be strain specific and is not solely related to the level of sensitivity/resistance to the specific compounds. Thus, the compounds of the present invention are intended to be useful on all bacterial strains including those not mentioned herein.

Preferably, the bacteria to be inhibited by administration of the pharmaceutical composition according to the present invention are bacteria that are resistant to beta-lactam antibiotics. More preferably, the bacteria to be inhibited are beta-lactamase positive strains that are highly resistant to beta-lactam antibiotics. The terms "resistant" and "highly resistant" are well-known by those of ordinary skill in the art.

Polymicrobial infections often include pathogens that produce beta-lactamase enzymes. These enzymes commonly cause resistance to penicillins and cephalosporins. Without treatment these microbes would multiply and thrive unimpeded, with serious or critical consequences to the patient.

Thus the present invention also relates to methods for overcoming bacterial antibiotic resistance.

In particular, the compounds of the general formula (Ia) and (Ib) of the present invention exhibit pharmacological activity and are therefore useful as pharmaceuticals.

Representative bacterial infections that can be treated using the antibiotic with inherent beta-lactamase inhibitory activity of formula (I), in particular of formula (Ia) or (Ib), of the invention include, but are not limited to, bacterial infections caused by bacteria of the genus Pasteurella, Haemophilus, Fusobacterium, Moraxella, Bacteroides, Aeromonas, Escherichia, Enterobacter, Klebsiella, Salmonella, Shigella, Serratia, Ureaplasma, Chlamydia, Actinobacillus, Streptococcus, Edwardsiella, Staphylococcus, Enterococcus, Bordetella, Proteus, Mycoplasma, or Mannheimia.

Compounds of the present invention of the general formula (I), e.g. of formula (Ia) and (Ib), in particular of formula (Ia), are therefore suitable for the treatment and prevention of diseases which are mediated by microbes, e.g. by bacteria. Representative bacterial infections that can be treated using the antibiotic with inherent beta-lactamase inhibitory activity of formula (I), in particular of formula (Ia) or (Ib), of the invention include, but are not limited to, bacterial infections caused by Pasteurella haemolytica, Pasteurella multocida, Pasteurella haemolytica, Haemophilus somnus, Actinobacillus pleuropneumoniae, Actinomyces pyogenes, Pseudomonas aeruginosa, Klebsiella pneumonia, Klebsiella oxytoca, Escherichia coli, Staphylococcus aureaus, Staphylococcus intermedius, Enterococcusfaecalis, Enterococcus faecium, Streptococcus pyogenes, Bacillus subtilis, Peptococcus indolicus, Mycoplasma bovis, Mycoplasma dispar, Mycoplasma hyopneumoniae, Mycoplasma hyorhinis, Mycoplasma gallisepticum, Mycoplasma mycoides, Mycoplasma ovipneumonia, Haemophilus influenzae, Klebsiella salmonella, Shigella, Proteus enterobacter, Enterobacter cloacae, Mannhemia haemolytica, Haemophilus somnus, Fusobacterium necrophorum, Bacterioides melaninogenicus, Proteus mirabïllis, Streptococcus suis, Salmonella cholerasuis, Edwardsiella ictaluri, Aeromonas salmonicidia, Actinobaccilus pleuropneumoniae, and Bordetella bronchoseptica.

### Method of treatment

The compound of formula (I) and the pharmaceutical composition according to the present invention are useful for inhibiting bacterial growth in a variety of contexts. In a preferred embodiment of the present invention, the pharmaceutical composition according to the present invention is administered to an experimental cell culture *in vitro* to prevent the growth of beta-lactam resistant bacteria. According to another preferred embodiments the pharmaceutical composition according to the present invention is administered to an animal, including a human, to prevent the growth of beta-lactam resistant bacteria *in vivo*. The method according to this embodiment comprises administering a therapeutically effective amount of a pharmaceutical composition according to the present invention for a therapeutically effective period of time to an animal, including a human.

Thus, the present invention is also directed towards a method of inhibiting beta-lactamase comprising contacting the beta-lactamase with an effective amount of antibiotic with inherent beta-lactamase inhibitory activity of formula (I), in particular of formula (Ia) or (Ib), defined above.

According to a further embodiment the present invention provides a method of treatment of a bacterial infection in a human or animal subject wherein the method comprising administering to the subject in need thereof a therapeutically effective amount of antibiotic with inherent beta-lactamase inhibitory activity of formula (I) as defined above.

Thus, the present invention is also directed towards the use of a pharmaceutical composition as described above for the treatment of an infection in humans or animals caused by a bacterium.

The present invention is also directed to the use of a pharmaceutical composition as disclosed above for the treatment of a bacterial infection in humans or animals.

Additionally, the present invention is directed to a method of treating a bacterial infection in humans or animals comprising administering to a patient in need of such treating a therapeutically effective amount of the composition according to the present invention and at least one pharmaceutically acceptable excipient.

Finally, the present invention is also directed to the use of a therapeutically effective amount of the composition according to the present invention and at least one pharmaceutically acceptable excipient for the preparation of a medicament for treating a bacterial infection, preferably wherein said medicament is to be administered to a patient in need thereof.

Safe and effective dosages for different classes of patients and for different disease states will be determined by clinical trial as is required in the art. The specific dosage and treatment regimens for any particular patient will depend upon a variety of factors, including the activity of the specific compound employed, the age, body weight, general health status, sex, diet, time of administration, route and frequency of administration, rate of excretion, drug combination, the sensitivity of the pathogen to the particular compound selected, the virulence of the infection, the severity and course of the disease, and the patient's disposition to the disease. Such matters, however, are left to the routine discretion of the physician according to principles of treatment well known in the antibacterial arts.

The terms "therapeutically effective amount" and "therapeutically effective period of time" are used to denote known treatments at dosages and for periods of time effective to show a meaningful patient benefit, i.e., healing of conditions associated with bacterial infection, and/or bacterial drug resistance. Preferably, such administration should be parenteral, oral, sublingual, transdermal, topical, intranasal, intratracheal, or intrarectal. When administered systemically, the therapeutic composition is preferably administered at a sufficient dosage to attain a blood level of antibiotic with inherent beta-lactamase inhibitory activity of formula (I) of at least about 0.1 mg/mL, more preferably about 1 mg/mL, and still more preferably about 10 mg/mL. For localized administration, much lower concentrations than this may be effective, and much higher concentrations may be tolerated.

The pharmaceutical compositions according to the present invention for human delivery per unit dosage, whether liquid or solid, comprise from about 0.01% to as high as about 99% of antibiotic with inherent beta-lactamase inhibitory activity of formula (I) or derivative thereof, such as a salt or ester. The preferred range being from about 10 to about 60% and from about 1% to about 99.99% of one or more other compounds such as those discussed herein, preferably from about 40% to about 90%.

The pharmaceutical composition will generally contain from about 1 mg to about 2.0 g of the antibiotic with inherent beta-lactamase inhibitory activity of formula (I) or derivative thereof, such as a salt or ester. However, in general, it is preferable to employ dosage amounts in the range of from about 1 mg to 1000 mg and from about 50 mg to about 5 g of the other antibiotics discussed herein; preferably from about 250 mg to about 2000 mg.

In parenteral administration, the unit dosage will typically include the pure antibiotic with inherent beta-lactamase inhibitory activity of formula (I) in sterile water solution or in the form of a soluble powder intended for solution, which can be adjusted to neutral pH and isotonic. For children, a dose of about 5-25 mg/kg of body weight given 2, 3, or 4 times per day is preferred; a dose of 10 mg/kg is typically recommended.

Although illustrative embodiments of the invention have been described in detail, it is to be understood that the present invention is not limited to those precise embodiments, and that various changes and modifications can be effected therein by one skilled in the art without departing from the scope and spirit of the invention as defined by the appended claims.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

The invention is further described in connection with the following non-limiting examples regarding synthesis and testing of the compounds.

### EXAMPLES

### EXAMPLE 1:

### Ethyl 2-benzamidomethyl-3-oxo-4,4,4-trifluorobutanoate (1a)

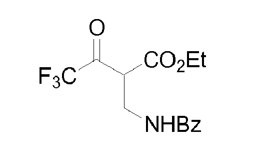

In a 1 I flask equipped with a dropping funnel and CaCl₂-tube are placed powdered sodium (10.0 g, 0.435 g. atom) and dry ether (350 mL). The suspension is cooled on an ice-bath and a solution of ethyl 4,4,4,-trifluoroacetoacetate (63 mL, 0.43 mol) in dry ether (100 mL) is added over 30 min. The mixture is stirred at rt until all sodium reacted (6 h). Then *N*-(chloromethyl)benzamide (72.8 g, 0.43 mol; prepared according to literature procedure H Bohme et al, Chem. Ber. 1959, 92, 1599-1607) is added portionwise and the mixture is stirred at rt for 30 min. The yellow colored suspension is diluted with EtOAc (250 mL) and filtered through short path of silica gel (20 g). Additional EtOAc (2 × 100 mL) is used to wash the product from silica gel. The filtrate is concentrated and the residue crystallized from cold (0°C) toluene (130 mL). The crystals are filtered off, rinsed successively with cold toluene (100 mL), toluene/*i*-Pr₂O (1:1, 100 mL) and *i*-Pr₂O (100 mL) yielding 70.99 g of the product as white crystals. An additional crop (18.3 g) is obtained after concentration of the filtrate and recrystallization of the residue from toluene (50 mL). The crystals are treated in the same manner as above. Total yield: 98.2 g, 72%. Mp 85-88 °C; ¹H NMR (300 MHz, CDCl₃/TMS) δ 1.27 and 1.30 (3H, 2t, *J* 7.2 Hz), 3.16 (0.2H, t), 4.01 (1.6H, t, *J* 6.1 Hz), 4.25 (1.5H, q, *J* 7.1 Hz), 4.39 (0.5H, t, *J* 6.1 Hz), 3.75-4.5 (1H, m), 5.19 (0.2H, s), 6.00 (0.2H, s), 6.69 and 6.74 (1H, 2 br t), 7.41-7.55 (3H, m), 7.71-7.76 (2H, m); ¹⁹F NMR (CDCl₃/CCl₃F) δ -66.94 (s), -78.3 (s), -85.3 (s); HRMS C₁₄H₁₄F₃NO₄ *m*/*z* (EI) calcd 317.0875, found 317.0877.

### EXAMPLE 2:

### Ethyl 2-benzamidomethyl-3-oxo-4-fluoro-butanoate (1b)

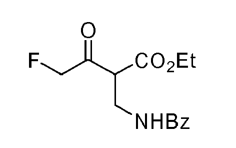

In a 2 L flask are placed ethyl γ-fluoroacetoacetate (47.1 g, 317 mmol) and THF (1.5 l) under inert atmosphere. The reaction mixture is cooled on an ice bath and LiH (3.79 g, 346 mmol) is added and left to stir for 15 minutes then left to warm up to room temperature over 45 min. *N*-(Chlorometil)benzamide (48.5 g, 286 mmol) is then added in one portion and after 15 min reaction mixture is poured onto sat. aq. NH₄Cl (2 L). Organic phase is collected and water phase extracted with EtOAc (1 L). Organic phases are combined and dried over MgSO₄. Solvent is evaporated and viscous residue left to solidify over night. Then the crude solid product is dispersed in a mixture of iPr₂O/Et₂O (6:1, 600 mlmL) for several hours and filtered. The process is repeated with filter cake obtained with a mixture of iPr₂O/Et₂O (2.2:1, 500 mlmL). Filter cake is eventually dispersed in iPr₂O (500 mlmL) so many times to achieve pure compound. White powder is collected yielding 31 g, 39%. Mp 76-77 °C; IR(KBr): 3306, 2989, 1744, 1716, 1638, 1528, 1297, 1259, 1204, 1088, 1032, 977, 696.¹H NMR (300 MHz, CDCl₃) δ 1.27 (3H, t, *J* 7.1 Hz), 3.82-4.10 (2H, m), 4.14 (1H, dt, *J* 2.6, 5.6 Hz), 4.22 (2H, q, *J* 7.2 Hz), 5.00 (2H, m), 6.89 (1H, m), 7.32-7.55 (3H, m), 7.65-7.85 (2H, m); ¹⁹F NMR (300 MHz, CDCl₃/CFCl₃) δ -227.45 (1F, dt, *J* 42.3, 3.1 Hz); MS (MH)⁺:282 *m*/*z*.

### EXAMPLE 3:

### Ethyl (2S,3S)-2-benzamidomethyl-3-hydroxy-4,4,4-trifluorobutanoate (2a)

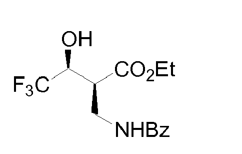

In a 2 L flask are placed β-amidomethyl δ-ketoester 1a (62.77 g, 198 mmol) in DMF (800 mL), a solution of Ru-complex prepared from [RuCl₂(C₆Me₆)]₂ (248 mg, 0.371 mmol) and (1*S*,2*S*)-*N*-(piperidyl-*N*-sulfonyl)-1,2-diphenylethylenediamine* (292 mg, 0.812 mmol) by heating in DMF (50 mL) at 80 °C for 30 min, and then HCO₂H-Et₃N 5:2 (50 mL). The solution is stirred at rt for 12 h. Water (1 L) is then added and the product extracted with ether (5 × 250 mL). The combined organic layers are washed with water (300 mL) and the aqueous layer reextracted with ether (3 × 500 mL). The combined ether layers are again washed with water (300 mL), dried over Na₂SO₄ and partially concentrated. The product that precipitated is filtered and washed with ether to obtain white crystals. An additional crop is isolated after concentrating the filtrate and washing the residue with *i*-Pr₂O. Total yield: 53.1 g, 84%, >99% ee, >99% de. %Ee is determined by HPLC analysis on Chiralcel OD column with hexane/*i*-PrOH 98:2 at a flow rate 1 mL/min and UV (227 nm) detection: t(*R*,*R*)= 51.8 min, t(*S*,*S*)= 56.3 min. %Ee can be also determined by ¹⁹F NMR with chiral shift reagent D-3-heptafluorobutyrylcamphorato praseodym(III) (1 mol equiv). %De is determined by ¹⁹F NMR: δ(syn) -77.53, δ(anti) -77.68. Mp 108-110 °C; [α]²⁵D +33.8 (*c* 1.0, CHCl₃); ¹H NMR (300 MHz, CDCl₃/TMS) δ 1.29 (3H, t, *J* 7.1 Hz), 2.96 (1H, dt, *J* 3.7, 9.3 Hz), 3.56 (1H, dt, *J* 3.4, 14.7 Hz), 4.22 (4H, m), 5.56 (1H, br d, *J* 5.4 Hz), 6.83 (1H, m), 7.42-7.59 (3H, m), 7.76-7.81 (2H, m); ¹⁹F NMR (CDCl₃/CCl₃F) δ -77.5 (d, *J* 7.1 Hz); Anal. Calcd for C₁₄H₁₆F₃NO₄: C, 52.67; H, 5.05; N, 4.39. Found C, 52.52; H, 5.11; N, 4.23.

### EXAMPLE 4:

### Ethyl (2S,3S)-2-benzamidomethyl-3-hydroxy-4-fluorobutanoate (2b)

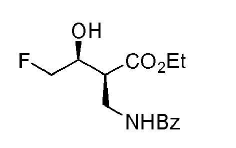

In a 500 mL flask [RuCl₂(1,3,5-triethylbenzene)]₂ (59.41 mg, 88.8 µmol) and (1S,2S)-N-(dimethylaminosulfonyl)-1,2-diphenylethylenediamine (62.46 mg, 195.5 µmol) in dry DMF (200 mL) are added and flushed with argon. The reaction mixture is heated to 80 °C for 40 min and then cooled to r.t.. Then α-amidomethyl β-ketoester 1b (10 g, 35.5 mmol) and HCO₂H-Et₃N 5:2 (8.9 mL) are added and left to stir at r.t. over night. Solvent is evaporated and crude product further purified with column chromatography to remove unwanted trans epimer (diastereoisomeric ratio for reaction is 80/20) (CH₂Cl₂/Et₂O 4:1). Colorless viscous liquid is obtained as pure sin diastereoizomer (6 g, 60%, 96% ee). %Ee is determined by HPLC analysis on Chiralcel OD column with hexane/*i*-PrOH/CF₃COOH 97:3:0.2 at a flow rate 1 mL/min and UV (222 nm) detection: t(*R*,*R*)= 44.5.min, t(*S*,*S*)= 58.3 min. [α]_{D}²⁶ 48.4, [α]₅₄₆²⁶ 57.0 (c 1.0, CHCl₃);¹H NMR (300MHz, CDCl₃) δ 1.30 (3H, t, *J* 7.1 Hz), 2.84 (1H, ddd, *J* 3.2, 4.0, 9.0 Hz), 3.57 (1H, m), 4.03 (1H, m), 4.14-4.27 (3H, m), 4.54.(1H, ddd, *J* 4.9, 10.6, 47.1Hz), 4.57.(1H, ddd, *J* 3.7, 9.8, 47.1Hz), 4.70 (1H, d, *J* 4.6 Hz), 6.81-6.96 (1H, m), 7.39-7.58 (3H, m), 7.71-7.83 (2H, m); ¹⁹F NMR (300MHz, CDCl₃/CFCl₃) δ -231.24 (1F, dt, *J* 20.3, 46.9 Hz). *m*/*z* (ESI) 284 *m*/*z* (M+H)⁺.

### EXAMPLE 5:

### (2S,3S)-2-Aminomethyl-3-hydroxy-4,4,4-trifluorobutanoic acid (3a)

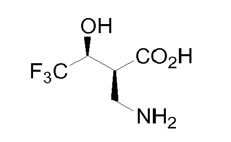

In a 3 L flask are placed alcohol 2a (85.5 g, 0.268 mol) and 10% HCl (1700 mL). The mixture is refluxed for 9.5 h under vigorous stirring. Cooling the reaction mixture on an ice-bath precipitats benzoic acid which is filtered off. The filtrate is washed with CH₂Cl₂ (2 × 250 mL, 100 mL) and the aqueous phase concentrated. The syrupy residue is dissolved in MeCN (150 mL), concentrated and further dried under high vacuum. The yellowish high viscosity syrup (66.2 g) is redissolved in MeCN (450 mL) and Et₃N (36.5 mL, 0.267 mol) is added. The mixture is stirred at rt overnight and the precipitate filtered off. The filter cake is suspended in CH₂Cl₂ (300 mL) and the mixture refluxed for 0.5 h, then filtered hot. The treatment of the filter cake with CH₂Cl₂ is repeated to remove all Et₃N-HCl salt. The product is obtained as white powder (38.4 g, 76.6%) containing 8% of epimer (2S,3R)-3a'. ¹H NMR (300 MHz, D₂O) δ 2.86 (1H, 'q', *J* 6 Hz), 3.31 (2H, d, *J* 6.1 Hz), 4.58 (1H, m); ¹⁹F NMR (D₂O/CCl₃F) δ -77.0 (d, *J* 7.6 Hz); *m*/*z* (FAB) 188 [100%, (M+H)⁺].

### EXAMPLE 6:

### (2S,3S)-2-Aminomethyl-3-hydroxy-4-fluorobutanoic acid (3b)

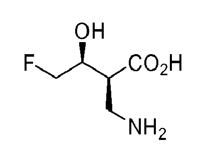

In a 500 mL flask equipped with a reflux condenser the alcohol 2b (6.00 g, 21.17 mmol) and 10% HCl (190 mL) are added and heated at reflux temperature under stirring for 4.5 h. Then the reaction mixture is cooled on ice bath and formed precipitate filtered off. Filtrate is washed with CH₂Cl₂ (4 × 25 mL) and water phase evaporated to dryness. The gummy residue is dried over night in vacuum over NaOH. Then the reddish viscous residue is suspended in MeOH (25 mL), cooled on an ice bath and NEt₃ (20.8 mmol, 2.80 mL) is added. After 15 min solvent is evaporated. Residue is dispensed in CH₂Cl₂ (150 mL), refluxed for 15 min and CH₂Cl₂ decanted. The process was repeated four times. Eventually gummy residue was dissolved in MeOH (17 mL) at 0°C and CH₂Cl₂ (87 mL) is added at once and solvent decanted. Gummy residue was dried in high vacuum to yield white amorphous solid (2.1 g, 65%). IR(KBr): 3411, 3270, 3179, 1518, 1401, 1347, 1110, 1052, 930, 875, 715; ¹H NMR (300MHz, D₂O) δ 2.60-2.75 (2H, m), 3.28 (1H, d, *J* 6.3 Hz), 4.07-4.29 (1H, m), 4.33-4.69 (2H, m); ¹⁹F NMR (300MHz, D₂O) δ -231.24 (1F, dt, *J* 22.4, 46.9 Hz); *m*/*z* (ESI) 150 *m*/*z* (M-H)⁻.

### EXAMPLE 7:

### (1'S,3S)-3-[1'-Hydroxy-2',2',2'-trifluoroethyl]-2-azetidinone (4a)

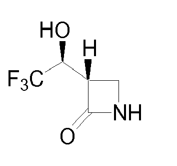

A 5 I three-necked flask equipped with an external stirrer, a reflux condenser and a thermometer is charged with β-amino acid 3a (31.20.4 g, 0.167 mol; containing 8% of epimer 3a'), MeCN (3 l), Ph₃P (48.8 g, 0.168 mol) and 2,2'-dipyridyl disulfide (37.0 g, 0.168 mol). The mixture is heated at 80 °C for 12 h. The solution is cooled down to rt and Cu(OAc)₂·H₂O (33.5 g) is added. The obtained orange precipitate is filtered off and the filtrate concentrated. The residue is suspended in toluene (300 mL) and the product extracted from toluene with water (7 × 250 mL). The combined aqueous layers are washed with toluene (100 mL) and then saturated with NaCl. The product is extracted with EtOAc (4 × 1 L), filtered through short path of silica gel/Na₂SO₄, and concentrated. The solid residue (22.9 g) is triturated with ether and the pure crystalline azetidinone 4a (15.65 g, 55.5%) is filtered off. The filtrate is concentrated and the residue purified by column chromatography on silica gel eluting with BuOAc. An additional amount of crystalline azetidinone 4a (1.38 g, 4.9%; *R*_{F} = 0.5 in EtOAc) is isolated after elution with BuOAc, concentration of appropriate fractions and treaturation of the residue with BuOAc. Further elution with EtOAc afforded epimeric azetidinone 4a' (0.90 g, 3.2%; *R*_{F} = 0.3 in EtOAc) as powder. (1'S,3S)-4a: mp 166-167 °C; [α]²⁵_{D} -42.1 (*c* 1.0, MeOH); ¹H NMR (300 MHz, CD₃OD/TMS) δ 3.29 (1H, 't', *J* 5.5 Hz), 3.50 (1H, m), 3.57 (1H, dt, *J* 5.4, 2.4 Hz), 3.34 (1H, dq, *J* 7.6, 2.2 Hz); ¹³C NMR (CD₃OD) δ 37.0, 51.9, 66.7 (q, *J* 32 Hz), 126.7 (q, *J* 282 Hz), 170.1; ¹⁹F NMR (CD₃OD/CCl₃F) δ -78.4 (d, *J* 7.6 Hz); Anal. Calcd for C₅H₇F₃NO₂: C, 35.51; H, 3.58; N, 8.28. Found C, 35.65; H, 3.68; N, 8.02. Epimer (1*S*',3*R*)- 4a': mp 125.5-127 °C; [α]²⁵_{D} -19.4 (*c* 1.0, MeOH); ¹H NMR (300 MHz, CD₃OD/TMS) δ 3.22 (1H, dd, *J* 5.6, 2.4 Hz), 3.41 (1H, t, *J* 5.6 Hz), 3.55 (1H, dt, *J* 5.6, 2.7 Hz), 4.26 (1H, dq, *J* 7.6, 5.9 Hz); ¹⁹F NMR (CD₃OD/CCl₃F) δ -77.6 (d, *J* 7.6 Hz); HRMS C₅H₇F₃NO₂ *m*/*z* (EI) calcd 170.0429, found 170.0423.

### EXAMPLE 8:

### (1'S,3S)-3-[1-hydroxy-2-fluoroethyl]-2-azetidinone (4b)

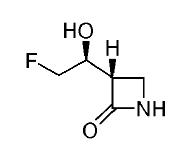

β-Amino acid 3c (2.00 g, 13.23 mmol), triphenylphosphin (4.16 g, 15.88 mmol) and 2,2'-dithiodipyridine (3.50 g, 15.88 mmol) are dissolved in dry DMSO (92 mL) under inert atmosphere. The reaction mixture is heated on an oil bath at 60°C for 4h. Than the reaction mixture is cooled on an ice bath and 2 mL of water is added. Solvents are distilled off in high vacuum.(T(bath)=60°C, p 0.05 mbar). The residue is further purified by column chromatography (toluene, then tolune/iPrOH/NEt₃ 4:1:0.1). Slightly yellowish crystals are obtained (740 mg, 42%). ¹H NMR (300 MHz, DMSO-d₆) δ 3.10-3.17 (2H, m), 3.18-3.25 (1H, m), 3.86-4.02 (1H, m), 4.17-4.52 (2H, m); ¹⁹F NMR: (300 MHz, CDCl₃/CFCl₃) δ -230.7 (1F, dt, *J* 19, 47 Hz). HRMS C₅H₈FNO₂ m/z (EI) calc 134.061732, found 134.061350.

### EXAMPLE 9:

### (1'S,3R,4R)-4-Acetoxy-3-[1'-hydroxy-2',2',2'-trifluoroethyl]-2-azetidinone (5a)

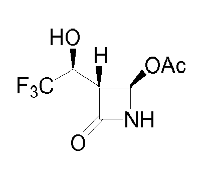

To a cooled solution (-10 °C) of azetidinone 4a (18.09 g, 0.107 mol), AcONa (8.78 g, 0.107 mol), and RuCl₃ ×H₂O (675 mg) in a mixture of AcOH (110 mL) and PrOAc (110 mL), a 16% solution of peracetic acid in PrOAc (167 g, 0.35 mol) is added dropwise at - 5 to 0 °C within 2 h. Then it is poured into 10% aq Na₂SO₃ (300 mL) and ether (250 mL) is added. The phases are separated and the aq phase extracted with ether (2 × 250 mL). The combined organic extracts are washed with satd aq NaHCO₃ (3 × 50 mL), and filtered through short path of silica gel/Na₂SO₄. The concentrated solid residue containing 5a and its epimer 5a' in dr = 90:10 is purified by silica gel column chromatography eluting with petroleum ether/ether 3:2, and treaturated with EtOAc/hexane to obtain acetoxyazetidinone 5a (15.9 g, 65.4%) in a crystalline form. *R*_{F} 0.67 (in ether); mp 110-114 °C; [α]²⁵_{D} +104.0 (*c* 1.0, CHCl₃); ¹H NMR (CDCl₃/TMS) δ 2.14 (3H, s), 3.56 (1H, 't', *J* 1.5 Hz), 4.17 (s, 1H,), 4.43 (1H, q, *J* 6.3 Hz), 6.00 (1H, s), 7.15 (1H, s); ¹⁹F NMR (CDCl₃/CCl₃F) δ -78.9 (d, *J* 6.9 Hz);.

### EXAMPLE 10:

### (1'S,3R)-4-Acetoxy-3-[1'-hydroxy-2-fluoroethyl]-2-azetidinone (5b)

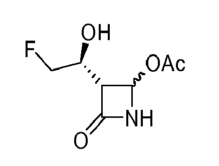

Azetidinone 4b (760 mg, 5.71 mmol), NaOAc anhydrous (3.50 g, 42.7 mmol) and RuCl_{3°}H₂O (111 mg, 0.534 mmol) are added to 1:1 mixture of AcOH:PrOAc (45 mL) and cooled to -40°C under argon. Then AcOOH (1.88 M in PrOAc, 13.6 mL) is added over 20 min. After the reaction mixture is warmed to -10°C it is vigorously stirred for 1h using external stirrer. Reaction mixture is filtered through a pad of silica gel and Celite^{®} eluting with CH₂Cl₂/EtOAc 1:1. The filtrate is dried (MgSO₄) and concentrated to yield red viscous liquid as mixture of epimers (80:20) (575 mg, 53%). ¹H NMR (300MHz, CDCl₃) δ 2.13 (2.3H, s), 2.18 (0.8 H, s)^{*}, 3.08 (1H, br s), 3.36 (0.8H, dd, *J* 1.3, 5.6 Hz), 3.60 (0.2H, ddd, *J* 2.0, 4.3, 9.9 Hz)^{*}, 4.20-4.35 (1H, m), 4.39-4.70 (2H, m), 5.89 (0.8H, d, *J* 0.9 Hz), 6.01 (0.2H, d, 4.3 Hz)^{*}, 6.66 (1H, s); ¹⁹F NMR (300MHz, CDCl₃/CFCl₃) δ - 230.3 (0.8F, dt, *J* 19.8, 47.0 Hz), 233.9 (0.2F, dt, *J* 21.7, 47.1 Hz)^{*}; ^{*}peaks correspond to other diastereoizomer; *m*/*z* (ESI) 209 (M+NH₄)⁺.

### EXAMPLE 11:

### (1'S,3R,4R)-4-Acetoxy-3-[2',2',2'-trifluoro-1'-trimethylsilyloxyethyl]-1-trimethylsilyl-2-azetidinone (5c)

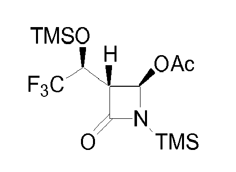

To a mixture of azetidinone 5a (11.30 g, 49.7 mmol) in dry CH₂Cl₂ (20 mL) is added *N*,*O*-bis(trimethylsilyl)acetamide (BSA, 30 mL). After being stirred at 40 °C for 2 h, the reaction mixture is concentrated and distilled on a Kugelrohr, first at 50 °C/0.1 mbar to remove the excess BSA and *N*-(trimethylsilyl)acetamide, then at 65 °C/0.1 mbar affording the product as a colorless oil (17.37 g, 94%). ¹H NMR (300 MHz, CDCl₃/TMS) δ 0.17 (9H, s), 0.29 (9H, s), 2.10 (3H, s), 3.53 (1H, t, *J* 1.3 Hz), 4.32 (1H, dq, *J* 1.2, 7.0 Hz), 6.33 (1H, s); ¹³C NMR (CDCl₃) δ 1.2, 1.9, 20.7, 57.6, 66.5 (q, *J* 33 Hz), 73.5, 124.1 (q, *J* 283 Hz), 164.1, 171.3; ¹⁹F NMR (CDCl₃/CCl₃F) δ -78.4 (d, *J* 6.2 Hz); HPLC-MS: *m*/*z* (M+H)⁺ 372.

### EXAMPLE 12:

### (1'S,3R,4R)-4-Acetoxy-3-[2',2',2'-trifluoro-1'-trimethylsilyloxyethyl]-2-azetidinone (5d)

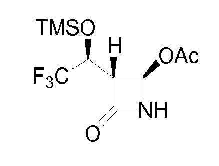

Protected azetidinone 6a (3.20 g, 8.6 mmol) is stirred with silica gel (1 g) in MeOH (20 mL) overnight. The reaction mixture is concentrated, and filtered through short path of silica gel eluting with EtOAc. The concentrated residue is distilled on Kugelrohr at 100 °C/0.1 mbar affording the product as colorless oil (2.50 g, 97%). ¹H NMR (300 MHz, CDCl₃/TMS) δ 0.17 (9H, s), 2.12 (3H, s), 3.52 (1H, t, *J* 1.4 Hz), 4.37 (1H, qd, *J* 6.7 1.4 Hz), 5.94 (1H, s), 6.60 (1H, br s); ¹⁹F NMR (CDCl₃/CCl₃F) δ -78.6 (d, *J* 7.0 Hz).

### EXAMPLE 13:

### (1'S,3R,4R)-4-Acetoxy-3-[2'-fluoro-1'-trimethylsilyloxyethyl]-2-azetidinone (5e)

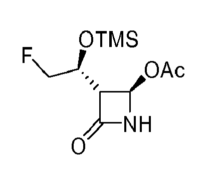

Azetidinone 5b (3.03 mmol, 580 mg) is dissolved in dry DMF (55 mL) under argon. Trimethylsilyl cyanide (15.2 mmol, 1.90 mL) is added and left to stir over night. Then the reaction mixture is flushed with argon (with outlet going to NaOH solution to neutralize HCN), excess reagent and solvent are removed by distillation (p=0.08 mbar, T=40°C). The resulting yellowish oily residue is dissolved in CH₂Cl₂ (0.1 mL/mg) and buffered silica gel is added and left to stir for 3 h. Reaction mixture is filtered and filtrate concentrated. If needed the residue is purified by flash chromatography (silica gel pH 8, eluting with a gradient of hexane/CH₂Cl₂) yielding pure product (710 mg, 57%).¹H NMR (300MHz, CDCl₃/CFCl₃) δ 0.14 (9H, s), 2.11 (3H, s), 3.33-3.40 (1H, m), 4.20-4.50 (3H, m), 5.87 (1H, s), 6.47 (1H, bs);¹⁹F NMR: (CDCl₃) δ -226.1 (1F, dt, *J* 17.6, 47.7 Hz).HRMS C₁₀H₁₈FNO₄SiNa (MNa⁺) *m*/*z*: 286.0886; Calculated: 286.0887;

### EXAMPLE 14:

### (3S,4R)-4-[(1R,3S)-3-Methoxy-2-oxo-cyclohexyl]-3-[2,2,2-trifluoro-1-(S)-trimethylsilyloxy-ethyl]-2-azetidinone (6a)

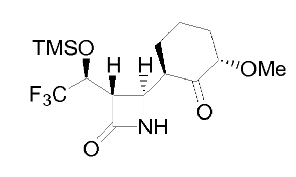

[(6*S*)-Methoxy-cyclohex-1-enolate]trimethylsilane: To a cold (0 °C) solution of *i*-Pr₂NH (62 mL, 0.44 mol) in dry THF (400 mL) is added drop-wise 2.0 M *t*-BuMgCl in ether (210 mL, 0.42 mol). After stirring at 0 °C for 1 h, (*S*)-2-methoxycyclohexanone (50 mL, 0.40 mol) is added over 2.5 h. The mixture is stirred for 1 h, then TMSCI (60 mL, 0.47 mol) is added, and the mixture was left to warm up to rt and stirred for 1 h. The white precipitate is filtered off, rinsed with ether and the filtrate partitioned between Et₂O (500 mL) and water (500 mL). The organic layer is washed with water (200 mL), dried (Na₂SO₄), and concentrated on rotary evaporator at 40 mmHg. The residue is distilled at 75-77 °C/12 mmHg affording colorless oil (65.83 g, 82.6%). ¹H NMR (300 MHz, CDCl₃/TMS) δ 0.19 (9H, s), 1.47-1.64 (3H, m), 1.87-2.09 (3H, m), 3.41 (3H, s), 3.50 (1H, t, *J* 3.0 Hz), 4.98 (1H, t, *J* 4.2 Hz).
Method A: To a cold (-60 °C) solution of [(6*S*)-methoxy-cyclohex-1-enolate]trimethylsilane (7.65 g, 38.2 mmol) in dry THF (70 mL) is added dropwise MeLi (1.5 M in ether, 24 mL) over 20 min. After stirring for 30 min at 0 °C, the lithium enolate is added to a cold (-78 °C) solution of azetidinone 5c (7.08 g, 19.0 mmol) in dry THF (70 mL). The reaction mixture is stirred at this temperature for 1 h then quenched with satd aq NH₄Cl (40 mL). The mixture is partitioned between EtOAc (40 mL) and water (40 mL), and the organic layer is dried (Na₂SO₄) and concentrated. The excess 2-methoxycyclohexanone is removed by distillation on Kugelrohr (70 °C/0.01 mmHg). The crude residue contained epimers 6a:6a' with dr = 82:18 and other imurities in about 20% amount. After purification by silica gel column chromatography (EtOAc/hexane 3:1) the product 8a is obtained as white crystals (3.50 g, 50%); further elution with EtOAc gave diastereomer 6a' (1.52 g, 22%). ¹H NMR (300 MHz, CDCl₃/TMS) δ 0.19 (9H, s), 1.67 (3H, m), 2.05 (2H, m), 2.25 (1H, m), 3.10 (1H, m), 3.25 (1H, t, *J* 2.4 Hz), 3.29 (3H, s), 3.59 (1H, t, *J* 3.0 Hz), 4.34 (1H, m), 4.40 (1H, dq, *J* 1.5, 7.3 Hz), 5.84 (1H, br s); ¹⁹F NMR (CDCl₃/CCl₃F) δ -78.3 (d, *J* 8.0 Hz); HRMS C₁₅H₂₅F₃NO₄Si *m*/*z* (M+H)⁺ calcd 368.1505, found 368.1514.
Method B: To a cold (-60 °C) solution of [(6*S*)-methoxy-cyclohex-1-enolate]trimethylsilane (1.08 g, 5.40 mmol) in dry THF (10 mL) is added dropwise MeLi (1.5 M in ether, 720 µl) over 20 min. After stirring for 30 min at 0 °C, the lithium enolate is added to a cold (-78 °C) solution of azetidinone 5d (800 mg, 2.67 mmol) in dry THF (5 mL). The reaction mixture is stirred at this temperature for 1 h then quenched with satd aq NH₄Cl (5 mL). The mixture is partitioned between EtOAc (10 mL) and water (10 mL), and the organic layer is dried (Na₂SO₄) and concentrated. The excess 2-methoxycyclohexanone is removed by distillation on Kugelrohr (70 °C/0.01 mmHg). The crude residue contained epimers 6a:6a' with dr = 82:18 is purified by silica gel column chromatography (EtOAc/hexane 3:1) yielding the product 6a as white crystals (706 mg, 72%).

### EXAMPLE 15:

### (3S,4R)-3-[(1S)-2-Fluoro-1-trimethylsilyloxy-ethyl]-4-[(1R,3S)-3-methoxy-2-oxo-cyclohexyl]-2-azetidinone (6b)

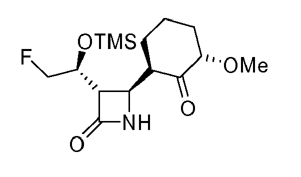

To a cold (-60 °C) solution of [(6S)-methoxy-cyclohex-1-enolate]trimethylsilane (616.3 mg, 3.08 mmol) in dry THF (10 mL) is added dropwise MeLi (1.6 M in ether, 1.9 mL) over 20 min. After stirring for 30 min at 0 °C, the lithium enolate is added to a cold (-100 °C) solution of azetidinone 5e (270 mg, 1.03 mmol) in dry THF (4 mL) over 40 min. The reaction mixture is stirred at this temperature for another 15 min and then quenched with sat. aq. NH₄Cl (20 mL). The mixture is extracted with 50 mL EtOAc and organic phase washed with saturated solution of NaHCO₃ (25 mL), brine (25 mL) and dried over MgSO₄. Solvent is evaporated and oily residue purified by flash chromatography (Hex/EtOAc 1:0 to 3:1) to yield pure diastereoisomer as white crystalline solid (120 mg, 35%). ¹H NMR (300MHz, CDCl₃) δ 0.15 (9H, s), 1.48-1.76 (3H, m), 1.91-2.16 (2H, m), 2.19-2.32 (1H, m), 2.98-3.12 (2H, m), 3.28 (3H, s), 3.55-3.60(1H, m), 4.03 (1H, dd, *J* 2.4, 5.1 Hz), 4.23-4.56 (3H, m), 5.80 (1H, bs); ¹⁹F NMR (300MHz, CDCl₃/CFCl₃) δ 225.4 (1F, dt, 17); *m*/*z* (ESI) 332 (M+H)⁺.

### EXAMPLE 16:

### (3S,4R)-4-[(1R,3S)-3-Methoxy-2-oxo-cyclohexyl]-3-[2,2,2-trifluoro-1-(S)-trimethylsilyloxy-ethyl]allyloxalyl-2-azetidinone (7a)

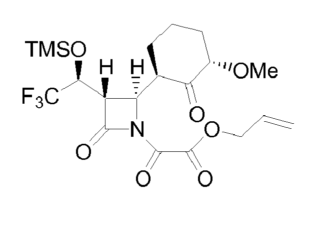

To a cold (0 °C) solution of 8a (1.85 g, 5.08 mmol) in DCM (15 mL) is added allyl oxalyl chloride (860 µl, 7.1 mmol) followed by Et₃N (1 mL, 7.6 mmol) over 10 min. The mixture is stirred at 0-5 °C for 1 h, then partitioned between water (50 mL) and CH₂Cl₂ (50 mL). The organic layer is washed with satd aq NaHCO₃ (30 mL), brine (30 mL), dried (Na₂SO₄), and concentrated. The residue is rapidly purified by silica gel column chromatography (hexane/EtOAc 4:1). Light yellowish crystals are obtained (2.30 g, 94.6%); mp 44-50 °C. ¹H NMR (300 MHz, CDCl₃/TMS) δ 0.16 (9H, s), 1.38 (1H, m), 1.70 (2H, m), 2.06 (2H, m), 2.23 (1H, m), 3.22 (3H, s), 3.53 (1H, m), 3.86 (1H, m), 3.97 (1H, dt, *J* 4.4, 13.2 Hz), 4.48 (2H, m), 4.80 (2H, m), 5.31 (1H, m), 5.40 (1H, m), 5.95 (1H, m); ¹⁹F NMR (CDCl₃/CCl₃F) δ -77.5 (1F, d, *J* 6.1 Hz); HRMS C₂₀H₂₉F₃NO₇Si *m*/*z* (M+H)⁺ calcd 480.1665, found 480.1670.

### EXAMPLE 17:

### (3S,4R)-3-[(1S)-2-fluoro-1-(trimethylsilyloxy)ethyl]-4-[(1R,3S)-3-methoxy-2-oxocyclohexyl]-allyloxalyl -2-azetidione (7b)

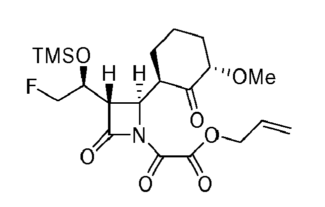

To a cold (0°C) solution of 6b (250 mg, 0.754 mmol) in dry CH₂Cl₂ (4 mL), allyl oxalyl chloride (1.05 mmol, 261 mg) and Et₃N (262 µL, 1.13 mmol) are added over 15 min and left to stir for 0.5 h. The reaction is filtered through pad of silica gel, rinsed with CH₂Cl₂.,and concentrated to obtain reddish viscous liquid (195 mg, 58%). ¹H NMR (300 MHz, CDCl₃) δ 0.12 (9H, s), 1.15-1.35 (2H, m), 1.48-1.76 (1H, m), 1.91-2.30 (3H, m), 3.24 (3H, s), 3.43 (1H, t, *J* 3 Hz) 3.54 (1H, t, *J* 3 Hz), 3.60-3.71 (1H, m), 4.27-4.52 (4H, m), 4.79-4.85 (2H, m), 5.23-5.49 (2H, m), 5.88-6.05 (1H, m); ¹⁹F NMR (300MHz, CDCl₃/CFCl₃) δ 224.0 (1F, dt, *J* 96, 10 Hz). HRMS C₂₀H₃₀FNO₇SiNa (MNa)⁺ 466.1687; calculated: 466.1673.

### EXAMPLE 18:

### Allyl (4S,8S,9R,10S)-4-methoxy-10-[2,2,2-trifluoro-1-(S)-trimethylsilyloxyethyl]-11-oxo-azatricyclo-[7.2.0.0^{3,8}]undec-2-ene-2-carboxylate (8a)

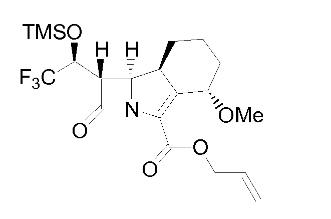

A solution of 7a (2.30 g, 4.80 mmol) and hydroquinone (250 mg, 2.27 mmol) in triethyl phosphite (15 mL) is heated at 140 °C for 2 h. Then it is cooled to rt and the excess P(OEt)₃ is distilled off on Kugelrohr (40 °C/0.1 mmHg). The residue is purified by silica gel column chromatography (hexane/EtOAc 9:1) to yield yellowish crystals (1.77 g, 58.8%). ¹H NMR (300 MHz, CDCl₃/TMS) δ 0.20 (9H, s), 1.22-1.59 (2H, m), 1.63 (1H, m), 1.86 (2H, m), 2.08 (1H, m), 3.25 (1H, m), 3.26 (3H, s), 3.54 (1H, m), 4.44 (2H, m), 4.70 (1H, dd, *J* 4.8, 12.9 Hz), 4.79 (1H, dd, *J* 4.5, 12.6 Hz), 5.27 (1H, d, *J* 10.2 Hz), 5.44 (1H, d, *J* 17.1 Hz), 5.96 (1H, m); ¹⁹F NMR (CDCl₃/CCl₃F) δ -78.5 (d, CF₃; *J* 8.2 Hz); HRMS C₂₀H₂₉F₃NO₅Si *m*/*z* (M+H)⁺ calcd 448.1767, found 448.1760.

### EXAMPLE 19:

### Allyl (4S,8S,9R,10S)-4-metoxy-10-[2'-fluoro-1-(S)-(trimethylsilyloxy)ethyl]-11-okso-azatricyclo[7.2.0.0^{3,8}] undec-2-ene-2-carboxylate (8b)

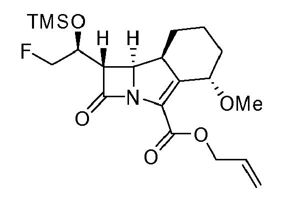

A solution of 7b (195 mg, 0.405 mmol), hydroquinone (8 mg, 0.73 mmol) and triethyl phosphite (0.493 mL, 2.38 mmol) is heated at 140°C for 4 h and then cooled to room temperature. The concentrated residue is purified by flash chromatography (gradient of EtOAc/Hexane 0:1 to 1:10). Colorless viscous liquid was obtained (110mg, 61%). ¹H NMR (300MHz, CDCl₃) δ 0.16 (9H, s), 1.29-1.51 (2H, m), 1.60-1.71 (1H, m), 1.75-1.93 (1H, m), 2.02-2.14 (m, 1H), 3.15-3.30 (4H, m), 3.37-3.42(1H, m), 4.19-4.48 (4H, m), 4.60-4.86 (2H, m), 4.96 (1H, *J* 3 Hz), 5.27 (1H, ddd, *J* 1.3, 2.6, 10.4Hz ), 5.44(1H, ddd, *J* 1.5, 3.0, 17.2Hz), 5.88-6.05 (1H, m); ¹⁹F NMR: (CDCl₃/CFCl₃) δ 227.2 (1F, dt, *J* 48 ,14 Hz); HRMS (M⁺) *m*/*z* 411.188350; calculated: 411.187725.

### EXAMPLE 20:

### Allyl (4S,8S,9R,10S)-4-methoxy-10-[1-(S)-hydroxy-2,2,2-trifluoroethyl]-11-oxo-azatricyclo[7.2.0.0^{3,8}] undec-2-ene-2-carboxylate (9a)

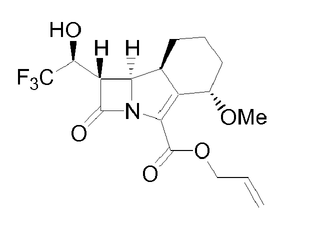

To a solution of 8a (1.63 g, 3.6 mmol) in THF (30 mL) is added a solution of AcOH (830 µl) and Bu₄NF (1M in THF, 10.9 mL) in THF (10 mL). After being stirred for 30 min, the reaction mixture is partitioned between EtOAc (100 mL) and satd aq NaHCO₃ (50 mL). The organic layer is further washed with NH₄Cl (50 mL), dried (Na₂SO₄), and concentrated to afford a white crystalline product (1.39 mg, 98%). Mp 78-85 °C; ¹H NMR (300 MHz, CDCl₃/TMS) δ 1.24 (2H, m), 1.51 (1H, m), 1.71 (2H, m), 1.94 (1H, m), 3.10 (4H, m), 3.43 (1H, m), 4.36 (1H, dd, *J* 3.0, 10.6 Hz), 4.43 (1H, dd, *J* 1.9, 6.7 Hz), 4.55 (1H, ddt, *J* 1.3, 5.7, 13.2 Hz), 4.66 (1H, ddt, *J* 1.5, 5.7, 13.2 Hz), 5.13 (1H, dq, *J* 1.4, 10.5 Hz), 5.26 (1H, dq, *J* 1.5, 17.1 Hz), 5.81 (1H, m); ¹⁹F NMR (CDCl₃/CCl₃F) δ -78.8 (d, *J* 6.7 Hz); *m*/*z* (FAB) 376 [75%, (M+H)⁺].

### EXAMPLE 21:

### Allyl (4S,8S,9R,10S)-4-metoxy-10-[2'-fluoro-1-(S)-hydroxyethyl]-11-oxo-azatricyclo-[7.2 .0.0^{3,8}]undec-2-ene-2-carboxylate (9b)

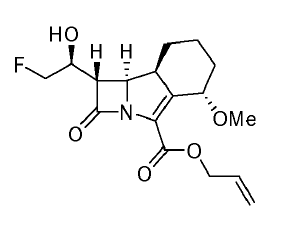

To a solution of 8b (105 mg, 0.255 mmol) in THF (2.5 mL) a solution of Bu₄NF (382 µL, 1 M in THF) and AcOH (29 µL, 0.51 mmol) are added dropwise. After being stirred for 30 min, the reaction mixture is partitioned between EtOAc (25 mL) and sat. aq. Na-HCO₃ (25 mL). The organic layer is further washed with sat. aq. NH₄Cl (25 mL) and the organic phase dried (MgSO₄). The concentrated residue is purified by flash chromatography (gradient EtOAc/Hex 0:1to 1:1). Colorless viscous liquid was obtained (52 mg, 60%). ¹H NMR (300MHz, CDCl₃) δ 1.31-1.51 (2H, m), 1.60-1.72 (1H, m), 1.76-1.96 (m, 2H), 2.03-2.15 (1H, m), 2.32 (1H, d, *J* 5Hz ), 3.21-3.34 (4H, m), 3.38 (1H, dd, *J* 3, 7Hz), 4.23-4.59 (3H, m), 4.62-4.89 (3H, m), 4.97 (1H, t, *J* 3.0 Hz), 5.28 (1H, ddd, *J* 1.3, 2.6, 10.5 Hz), 5.43 (1H, ddd, *J* 1.5, 3.0, 17 Hz), 5.90-6.05 (1H, m).¹⁹F NMR (300MHz, CDCl₃/CFCl₃) δ -232.2 (1F, dt, *J* 19, 47 Hz). HRMS C₁₇H₂₂FNO₅ (M⁺) m/z 339.148820; calculated: 339.148201.

### EXAMPLE 22:

### Allyl (4S,8S,9R)-4-methoxy-10-[(E)-(2,2,2-trifluoroethylidene]-11-oxo-azatricyclo[7.2.0.0^{3,8}]undec-2-ene-2-carboxylate (10a)

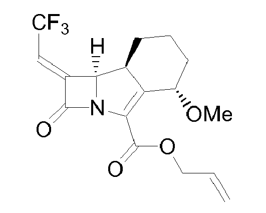

To a cold (0 °C) solution of 9a (250 mg, 0.67 mmol) in dry CH₂Cl₂ is added Ph₃P (175 mg, 0.67 mmol) and DEAD (1 equiv). After stirring at rt for 30 min, the reaction mixture is concentrated and the residue purified by silica gel column chromatography (eluting with CH₂Cl₂) to yield a light yellowish oil which crystallized upon standing (120 mg, 50%). ¹H NMR (300 MHZ, CDCl₃/TMS) δ 1.17 (1H, m), 1.51 (2H, m), 1.84 (2H, m), 2.09 (1H, m), 3.28 (3H, s), 3.37 (1H, m), 4.72 (1H, m), 4.84 (1H, m), 4.90 (1H, m), 5.00 (1H, m), 5.30 (1H, m), 5.45 (1H, m), 5.98 (1H, m), 6.37 (1H, dq, *J* 1.7, 7.6 Hz); ¹⁹F NMR (CDCl₃) δ -62.3 (d, *J* 8 Hz); HRMS C₁₇H₁₉F₃NO₄ *m*/*z* (M+H)⁺ calcd 358.1266, found 358.1273.

### EXAMPLE 23:

### Sodium (4S,8S,9R,10S)-4-methoxy-10-[1-(S)-hydroxy-2,2,2-trifluoroethyl]-11-oxo-azatricyclo[7.2.0.0^{3,8}]undec-2-ene-2-carboxylate (11a)

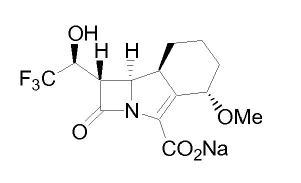

To a solution of 9a (0.94 g, 2.5 mmol) in dry THF (8 mL) is added a solution of Ph₃P (65 mg, 0.25 mmol) and Pd(PPh₃)₄ (72 mg, 0.06 mmol) in dry THF (8 mL) followed by sodium 2-ethylhexanoate (0.21 M in THF, 11.9 mL, 2.5 mmol). The reaction mixture is stirred for 2 h and the obtained precipitate filtered through Durapore^{®} HV (0.22 µ) filter and washed with THF (10 mL) and then pentane (10 mL) to afford (I)a as an off white powder (408 mg, 36.5%). Mp cannot be determined. Product started to darken at 220 °C. ¹H NMR (300 MHz, DMSO-d₆/TMS) δ 1.13-1.38 (2H, m), 1.46-1.73 (m, 3H), 1.83 (1H, m), 2.84 (1H, m), 3.09 (3H, s), 3.39 (1H, t, *J* 3.3 Hz), 4.09 (1H, dd, *J* 10.3, 3.1 Hz), 4.38 (1H, m), 5.09 (1H, t, *J* 2.8 Hz), 6.91 (1H, s); ¹⁹F NMR (DMSO-d₆/CCl₃F) δ -76.2 (d, *J* 8 Hz); HRMS (EI) *m*/*z* (M+H)⁺ calculated for C₁₄H₁₆F₃NNaO₅ 358.0878, found 358.0858.

### EXAMPLE 24:

### Sodium (4S,8S,9R,10S)-4-metoxy-10-[2'-fluoro-1-(S)-hydroxyethyl]-11-okso-azatricyclo-[7.2.0.0^{3,8}]undec-2-ene-2-carboxylate (11b)

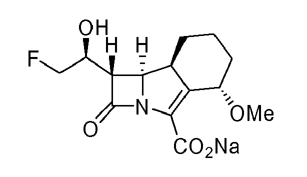

To a solution of 9b (36.0 mg, 0.106 mmol) in THF is added a solution of Pd(PPh₃)₄ (3.1 mg, 2.65 µmol) and PPh₃ (2.8 mg, 10.6 µmol) in THF (1 mL) under argon followed by sodium 2-ethylhexanoate (17.6 mg, 106 µmol). The mixture is left to stir at r.t. for 0.5h and formed solid filtrated trough PTFE filter (0.45 µm) and washed with THF (2 mL). White amorphous solid is obtained (15 mg, 44%). ¹H NMR (300MHz, DMSO-*d*₆) δ 1.05-1.37 (2H, m), 1.44-1.90 (4H, m), 2.75-2.90 (1H, m), 3.16 (1H, dd, 3, 6 Hz), 3.90-4.08 (2H, m) 4.32 (1H, ddd, *J* 5.5, 9.6, 47Hz), 4.38 (1H, ddd, *J* 3.6, 9.5, 48Hz), 5.07 (1H, t, *J* 3 Hz) 5.50 (1H, d, 5Hz).¹⁹F NMR (300MHz, DMSO-d₆/CFCl₃) δ: - 226.8 (1F, dt, *J* 48, 20.5 Hz). MS (ESI) *m*/*z* 300 (M+H)⁺.

### EXAMPLE 27:

### Sodium (4S,8S,9R)-4-methoxy-10-[(E)-2,2,2-trifluoroethylidene]-11-oxo-azatricyclo[7.2.0.0^{3,8}] undec-2-ene-2-carboxylate (12a)

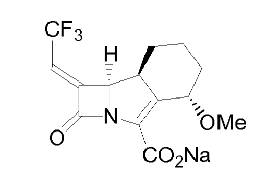

To a solution of 10a (100 mg, 0.28 mmol) in dry THF (2 mL) is added a solution of Ph₃P (7 mg, 0.1 equiv) and Pd(PPh₃)₄ (0.025 equiv) in dry CH₂Cl₂. Then a solution of sodium 2-ethylhexanoate (1 equiv) in dry THF (cca 1 mL) is added and stirred at rt for 3 h. The precipitate is filtered off using PTFE filter (0.45 µ) and then recrystallized from dry THF to afford the product as yellowish crystals (43 mg, 45%). ¹H NMR (300 MHz, DMSO-*d*₆) δ 0.92 (1H, m), 1.27 (1H, m), 1.54 (3H, m), 1.84 (1H, m), 3.01 (1H, m), 3.13 (3H, s), 4.79 (1H, m), 5.17 (1H, m); 6.78 (1H, dq, *J* 1.2, 7.8 Hz); ¹⁹F NMR (DMSO-*d*₆) δ -56.1 (d, *J* 8 Hz); HRMS C₁₄H₁₄F₃NNaO₄ *m*/*z* (M+H)⁺ calcd 340.0773, found 340.0766.

### TESTING EXAMPLES

### EXAMPLE 28

### Materials and Methods

### IC₅₀ Determination for the Inhibitors of beta-Lactamases of formula (I):

The IC₅₀ value represents the concentration of inhibitor required to effect a 50% loss of activity of free enzyme. A standard test for the production of beta-lactamase involves use of the chromogenic cephalosporin, nitrocefin. This compound exhibits a rapid distinctive colour change from yellow (maximum OD at pH 7.0 at lambda 390 nm) to red (maximum OD at pH 7.0, at lambda 486 nm), as the amide bond in the beta-lactam ring is hydrolysed by a beta-lactamase.

Homogeneously purified class A beta-lactamases TEM-1 and SHV-1 from *E*. *coli* and class C enzyme P99 from *Enterobacter cloaca* were employed in the assay.

All the enzymes and compounds were dissolved in 50 mM phosphate buffer pH 7.0 and all further dilutions were done with the same buffer solution. Enzyme and compound dilutions were pre-incubated for 30 min at 37°C and in a final volume of 500 µl. Than 10 µl of 5 mM nitrocefin (reporter substrate) was added to the solution and the absorbance at 482 nm was measured during 2 to 5 minutes. The initial rate was calculated for all the different solutions. The IC₅₀ values were determined as the inhibitor concentration that gave an initial hydrolysis rate of nitrocefin equal to 50 % of the hydrolysis rate of nitrocefin in absence of inhibitor.

Representative compounds of formula (I) were evaluated as inhibitors of beta-lactamases of TEM-1 and SHV-1 (class A, penicillinase) from *E*. *coli* and P-99 (class C, cephalosporinase) from *Enterobacter cloacae*, by relative IC₅₀ analysis using a procedure similar to that described above. The data are presented in Table 1 below.

**Table 1: In vitro IC₅₀ activities of inhibitors of beta-lactamases LK-180, LK-178 and tazobactam against class A (TEM-1 and SHV-1) and class C (P99) beta-lactamases.**

| | | IC₅₀ [µM] | | |
|---|---|---|---|---|
| Compound | R | TEM-1 | SHV-1 | P99 (AmpC) |
| tazobactam | | 0.017 | 0.222 | 1.808 |
| LK-178 | CF₃- | 8.400 | 1.100 | 0.419 |
| LK-180 | FCH₂- | 0.500 | 0.012 | 0.0005 |

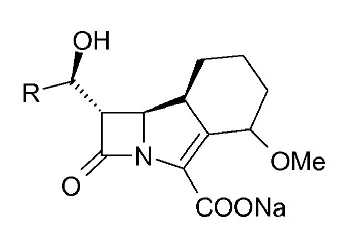

Sodium salt of (4*S*,8*S*,9*R*)-4-methoxy-10-(1-(S)-hydroxy-fluoroethyl)-11-oxo-1-azatricyclo-[7.2.0.0 (3,8)] undec-2-ene-2-carboxylic acid, (thereafter referred to as "LK-180") a pharmaceutically acceptable salt or ester thereof was selected from a series of inhibitors of beta-lactamases of formula (I) as the most promising inhibitor of beta-lactamases.

LK-180 displayed highly potentiated activity against SHV-1 and AmpC enzymes as compared to tazobactam which is used in combination with piperacillin (Tazocin^{®}) as golden standard to treat various bacterial infections in human and animals. Currently there are no inhibitor of class C enzymes available on the market.

### EXAMPLE 29

Antibiotic Effect of LK-180 when Tested Against Class A and Class C beta-Lactamase Positive Bacterial Strains in Broth Microdilution Assay.

Representative antibiotic with inherent beta-lactamase inhibitory activity of formula (I) was tested in microdilution susceptibility assay (Table 2).

### Antibiotics

Stock solutions of the test compounds and Tazocin^{®} were prepared in distilled water according to the CLSI guidelines [Methods for dilution antimicrobial tests for bacteria that grow aerobically. NCCLS document M7-A5; 2000; vol. 19. Clinical and Laboratory Standards Institute, Villanova, Pa.].

### Bacterial strains

All tested strains and clinical isolates were either purchased from the American Type Culture Collection (ATCC), or from the in-house company culture collection. The tested strains were purely used for the purposes of illustrative example, and are by no means essential for performing the invention.

Representative antibiotic with inherent beta-lactamase inhibitory activity of formula (I) was evaluated against the bacterial strains producing serine-based class A beta-lactamases including CTX-M, TEM-type, SHV-type extended spectrum beta-lactamases (ESBL) and class C beta-lactamases (AmpC) as noted in Table 2.

### Cultivation and Maintenance of Test Organisms

The strains were processed according to procedures recommended by ATCC, or procedures that are routinely used. Frozen bacterial stocks were thawed to room temperature, and a few drops were placed on an appropriate blood agar plate. The cultures were subcultured on a fresh Mueller-Hinton agar plate (MHA) the following day, and the subcultures were again incubated overnight.

### Inoculum

Bacterial suspensions with a turbidity equivalent to that of a 0.5 McFarland standard were prepared by suspending a tiny portion of one colony from blood agar plates in 2 mL of sterile saline. Suspensions were further diluted with cation adjusted Mueller Hinton Broth (CAMHB) to obtain a final inoculum of 5 x 10⁵ CFU/ mL.

### Assay Procedure

The *in vitro* activities of the antibiotics were determined by the broth microdilution method as recommended by CLSI guidelines.

MIC experiments were performed in duplicate in 96-well microtiter plates using CAMHB. Serial twofold dilutions of each antibiotic were prepared in CAMHB. The bacterial suspension with final inoculum 10⁵ CFU/mL was transferred to the test medium containing the antibacterial substances. In each well of microtiter plate 50 µL of bacterial inoculum and 50 µL of antibiotic dilutions were combined. Each plate included 4 wells with no bacterial inoculum (negative control) and 4 wells with no test compound and no antibiotic (positive control). The plates were incubated at 35°C for 24 h. Purity check and colony counts on each inoculum suspension was performed to ensure that the final inoculum concentration routinely obtained closely approximates 5 x 10⁵ CFU/ mL.

In addition the assay is routinely monitored by testing standard antibiotics and ensuring that MIC values are within the recommended ranges for the respective control strains.

The minimal inhibitory concentration (MIC) for all isolates was defined as the lowest concentration of antimicrobial agent that completely inhibits the growth of the organism as detected by the unaided eye.

**Table 2. MIC values of LK-180 are compared to Tazocin^{®}.**

| | **MIC (mg/L)** | | | | | | | | | |
|---|---|---|---|---|---|---|---|---|---|---|
| **Compound** | **S1** | **S2** | **S3** | **S4** | **S5** | **S6** | **S7** | **S8** | **S9** | **S10** |
| LK-180 | 8 | 8 | 64 | 16 | 4 | 8 | 4 | 2 | 16 | 128 |
| | 4 | 4 | 64 | 16 | 4 | 8 | 4 | 2 | 16 | 128 |
| Tazocin^{®} | 64 | 64 | 64 | 16 | 4 | 16 | 8 | 16 | 16 | 32 |
| | 64 | 32 | 64 | 8 | 4 | 16 | 8 | 16 | 16 | 32 |
| | | | | | | | | | | |

| **Compound** | **S11** | **S12** | **S13** | **S14** | **S15** | **S16** | **S17** | **S18** | **S19** | **S20** |
|---|---|---|---|---|---|---|---|---|---|---|
| LK-180 | 8 | 4 | 4 | 4 | 32 | 16 | 8 | 32 | 8 | 8 |
| | 8 | 4 | 4 | 4 | 32 | 16 | 8 | 32 | 8 | 8 |
| Tazocin^{®} | 64 | 16 | 32 | 16 | 256 | 16 | 32 | 64 | 32 | 128 |
| | 64 | 16 | 16 | 16 | 128 | 16 | 32 | 64 | 16 | 64 |

**Table 3. List of bacterial strains used in microdilution susceptibility assay.**

| ID | Bacterial strain | No. | beta-lactamase |
|---|---|---|---|
| S1 | *E. cloacae* | Lek 24 | AmpC |
| S2 | *E. cloacae* | Lek 14, derepressed | AmpC |
| S3 | *E. cloacae* | 4013 | *not defined* |
| S4 | *E.* caerogenes | ATCC 29751 | class II |
| S5 | *E. faecalis* | ATCC 29212 | *not defined* |
| S6 | *K. pneumoniae* | B312 | SHV-18 |
| S7 | *K. pneumoniae* | DSA 1461 | *not defined* |
| S8 | *K. pneumoniae* | ATCC 700603 | SHV-18 |
| S9 | *C. freundii* | B271 | SHV-5 |
| S10 | *C. freundii* | B318 | CTX-M |
| S11 | *E. cloacae* | Lek 30, derepressed | AmpC |
| S12 | *E. coli* | B308 | *not defined* |
| S13 | *E. coli* | B309 | *not defined* |
| S14 | *E. faecalis* | Lek 112 | *not defined* |
| S15 | *E. faecium* | Lek 116 | *not defined* |
| S16 | *K. pneumoniae* | Lek 8 | *not defined* |
| S17 | *K. pneumoniae* | B401 | TEM-10, TEM-12 |
| S18 | *K. pneumoniae* | Lek 22 | *not defined* |
| S19 | *C. freundii* | Lek 17 | AmpC |
| S20 | *C. freundii* | Lek4, derepressed | AmpC |

It can be shown by the assay described herein that compound LK-180 is superior to Tazocin^{®} against several ceftazidime-resistant strains tested. Overall LK-180 consistently expressed potentiated spectrum of activity against class A beta-lactamases including CTX-M, TEM-type, SHV-type extended spectrum beta-lactamases (ESBL) and class C beta-lactamases (AmpC) producing strains.

Thus LK-180 represents high potential for further development as antibiotic and beta-lactamase inhibitor.

### References cited in the application

Bush K; et al; Antimicrob. Agents Chemother. 1995, 39 (6): 1211-1233; Thomson KS; et al; Microbes and Infections 2000, 2: 1225-1235
Li et al., Antimicrob Agents Chemother 1995, 39:1948-1953
Livermore, DM. J. Antimicrob. Chemother. 1998, 41 (D), 25-41
Livermore, DM. Clinical Microbiology Reviews 1995, 8 (4), 557-584
Helfand MS; et al; Curr. Opin. Pharmacol. 2005, 5: 452-458
BuynakJD. Curr. Med. Chem. 2004, 11, 1951-1964
Bonnefoy A et al, J. Am. Chem. Soc. 2004, 54, 410-417
Weiss WJ et al; Antimicrob. Agents Chemother. 2004, 48, 4589-4596
Phillips OA at al; J. Antibiot. 1997, 50, 350-356
Jamieson CE et al; Antimicrob. Agents Chemother. 2003, 47, 1652-1657
SK Spangler, et al, Antimicrob. Agents Chemother. 1997, 41, 1, 148-155
Cahn. Ingold and Prelog, Experientia 156, 12, 81. PROTECTIVE GROUPS in ORGANIC CHEMISTRY,T. W. Green, P.G. Wuts; John Wiley & Sons 1999
H Bohme et al, Chem. Ber. 1959, 92, 1599-1607
Methods for dilution antimicrobial tests for bacteria that grow aerobically. NCCLS document M7-A5; 2000; vol. 19. Clinical and Laboratory Standards Institute, Villanova, Pa.

## Claims

1. A compound of formula (I) 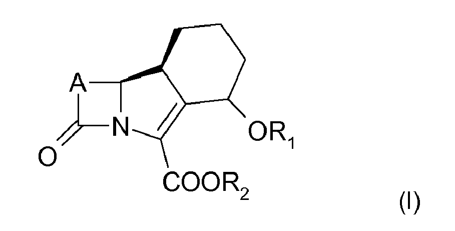 wherein A is defined as 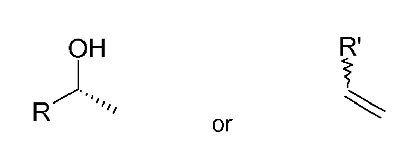 and wherein
R represents a C₁₋₂₀ fluorine- or chlorine-containing hydrocarbon moiety, straight, branched or cyclic, where the hydrocarbon moitey can be optionally interrupted or substituted by unsaturations;
R' represents a C₁₋₂₀ fluorine- or chlorine-containing hydrocarbon moiety, straight, branched or cyclic, where the hydrocarbon moitey can be optionally interrupted or substituted by unsaturations;
R¹ represents a hydrogen atom, a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon);
R² represents: a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

2. The compound according to claim 1, wherein the compound of formula (I) is a compound of the formula (Ia)

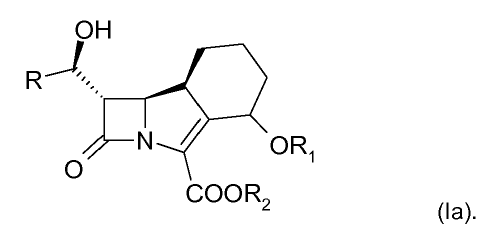

3. The compound according to claim 1, wherein the compound of formula (I) is a compound of the formula (Ib)

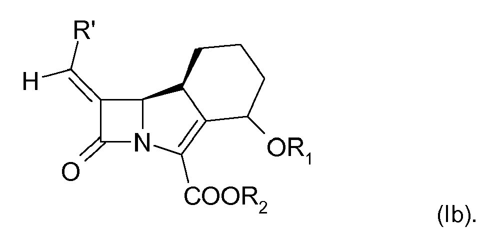

4. The compound according to any of claims 1 to 3, wherein in formula (I), R represents a CFH₂ group, a CF₂H group or a CF₃ group, and/or R¹ represents a methyl group.

5. A pharmaceutical composition at least comprising one compound according to any of claims 1 to 4.

6. A pharmaceutical composition according to claim 5, wherein the pharmaceutical composition additionally comprises a pharmaceutically acceptable carrier.

7. A pharmaceutical composition according to any of the claims 5 or 6, wherein the pharmaceutical composition additionally comprises a pharmaceutically acceptable excipient.

8. A compound of the general formula (Ia) 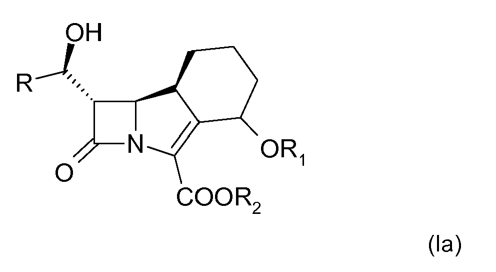 wherein
R represents a CFH₂ group, a CF₂H group or a CF₃ group,
R¹ represents a methyl group, and
R² represents: a hydrogen atom, a cation of metal group I or II (such as Li⁺, Na⁺, K⁺, Cs⁺, Ca²⁺, Mg²⁺), an ammonium or a C₁₋₂₀ quaternary ammonium (such as tetrabutylammonium) or a protonated form of a C₁₋₂₀ amine (such as trimethylamine, triethylamine, diisopropylethylamine, diethylisopropylamine, N,N,N',N'-tetramethylethylenediamine, N,N'-dibenzylethylenediamine, ethanolamine, diethanolamine, triethanolamine, dicyclohexylamine, piperidine, 1,8-diazabicyclo[5.4.0]undec-7-ene, 1,5-diazabicyclo[4.3.0]non-5-ene, 3,3,6,9,9-pentadimethyl-2,10-diazabicyclo[4.4.0]dec-1-ene, guanidine, cyanoguanidine, pyridine, 4-dimethylaminopyridine, imidazole, and the like), a C₁₋₂₀ hydrocarbon moiety, straight, branched or cyclic; wherein the hydrocarbon moitey can be optionally interrupted or substituted by unsaturations, C₅₋₆ arenes, heteroatoms (such as fluorine, chlorine, nitrogen, oxygen, sulfur, silicon).

9. A compound according to claim 8, wherein
- R represents a CH₂F group, and
- R² represents a hydrogen atom.

10. A process for preparing a compound of general formula (I) according to claim 1, comprising the steps of:
(a) asymmetric reduction of a compound of general formula (II) to obtain a compound of general formula (III): 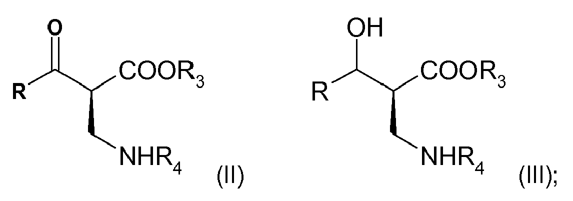
(b) cyclisation reaction to obtain a compound of general formula (IV): 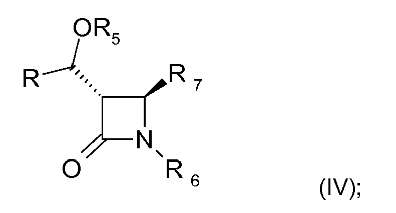
(c) preparation of a compound of general formula (V): 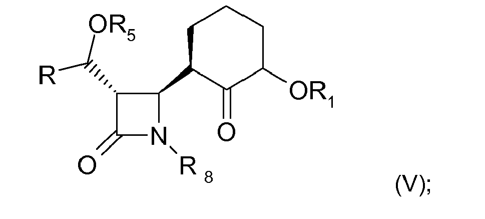
(d) cyclisation reaction to obtain a compound of general formula (VI):

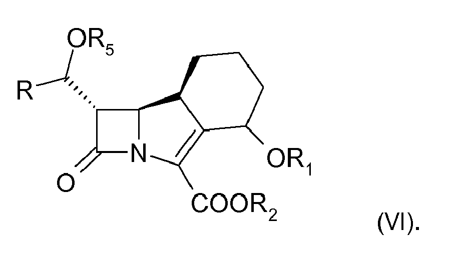

11. A therapeutically effective amount of one or more compounds of formula (I) as defined within any of claims 1 to 4 or a compound of formula (Ia) as defined in any of claims 8 or 9 or of a compound obtained by a process according to claim 10 for use as a broad-spectrum beta-lactamase inhibitor.

12. A therapeutically effective amount of one or more compounds of formula (I) as defined within any of claims 1 to 4 or a compound of formula (Ia) as defined in any of claims 8 or 9 or of a compound obtained by a process according to claim 10 for use as an antibiotic.

13. The therapeutically effective amount of one or more compounds of formula (I) as defined within any of claims 1 to 4 or a compound of formula (Ia) as defined in any of claims 8 or 9 or of a compound obtained by a process according to claim 10 for use as an antibiotic according to claim 12, wherein the antibiotic is a broad-spectrum antibiotic with beta-lactamase inhibitor activity, in particular against beta-lactamases of class A, C and D.

14. A pharmaceutical composition as claimed within any of the claims 5 to 7 or a compound as claimed in any of claims 1 to 4, 8 or 9 or a compound obtained by a process according to claim 10 for the treatment of a bacterial infection in humans or animals.

15. Use of a therapeutically effective amount of the composition according to any of claims 5 to 7 or a compound according to any of claims 1 to 4, 8 or 9 or a compound obtained by a process according to claim 10 and at least one pharmaceutically acceptable excipient for the preparation of a medicament for treating a bacterial infection, preferably wherein said medicament is to be administered to a patient in need thereof.

16. A method of treating a bacterial infection in humans or animals comprising administering to a patient in need of such treating a therapeutically effective amount of the composition according to any of claims 5 to 7 or a compound according to any of claims 1 to 4, 8 or 9 or a compound obtained by a process according to claim 10 and at least one pharmaceutically acceptable excipient.
